# EUROPEAN PATENT SPECIFICATION

(11) **EP 4 210 630 B1**
(45) Date of publication and mention of the grant of the patent: **29.10.2025**
(21) Application number: 21791083.5
(22) Date of filing: 08.09.2021
(51) Int. Cl.: A61F 2/12

(54) **SYSTEM FOR SCARLESS MASTOPEXY AND METHOD OF MAKING SAID SYSTEM**
SYSTEM FÜR NARBENFREIE MASTOPEXIE UND VERFAHREN ZUR HERSTELLUNG DIESES SYSTEMS
SYSTÈME POUR MASTOPEXIE SANS CICATRICE ET MÉTHODE DE FABRICATION DUDIT SYSTÈME

(30) Priority: 09.09.2020 US 202063076182 P
(43) Date of publication of application: 19.07.2023
(60) Divisional application: 25204286.6
(73) Proprietor: Tepha, Inc., Lexington MA 02421 (US)
(72) Inventor: LIMEM, Skander, Lynnfield, MA 01940 (US); VAN NATTA, Bruce, Lexington, MA 02421 (US); WILLIAMS, Simon F., Lexington, MA 02421 (US)
(74) Representative: Hoffmann Eitle
(86) International application number: PCT/US2021/049418
(87) International publication number: WO 2022/055966

(56) References cited:
- FR-A1- 2 972 916
- US-A1- 2010 137 679

## Description

This application claims the benefit of U.S. Provisional Application No. 63/076,182, filed September 9, 2020.

### FIELD OF THE INVENTION

The present invention generally relates to implants for mastopexy that can be used to lift tissues and organs in a minimally invasive manner, and in particular implants that can be used in plastic surgery procedures. The implants can be used to minimize the formation of scars during lifting procedures.

### BACKGROUND OF THE INVENTION

Numerous plastic surgery procedures are performed each year to restore or correct the form or function of the body. Many of these procedures seek to restore a youthful appearance, or even to enhance one's existing appearance. Natural factors, such as aging and gravity, contribute to the loss of the youthful appearance. For example, skin laxity, loss of muscle tone, and attenuation of ligaments can result in ptosis (drooping) of the breast. Plastic surgeons have developed a plethora of surgical techniques to correct the ptosis of different anatomical structures that occurs with aging. These techniques vary in the type of incision, direction of incision, plane of dissection, amount of dissection, extent of repositioning of tissue, the use of different types of sutures, different suturing techniques, and different fixation techniques.

Plastic surgeons have developed a number of different mastopexy procedures for lifting the breast. These procedures can, however, be very invasive, require extensive dissection, and can leave the patient with visible scars. For example, the lollipop or vertical mastopexy procedure is performed by making incisions around the areola, and a vertical incision in the lower pole of the breast from the areola to the inframammary fold (IMF). The anchor or Wise procedure is an even more invasive procedure than the lollipop procedure where an incision is made across the inframammary fold in addition to the vertical incision used in the lollipop procedure. Both the lollipop and anchor procedures can leave permanent scars on the lower pole of the patient's breast, and in that respect can provide poor cosmetic outcomes.

Less invasive suture-based mastopexy procedures have been developed which help to minimize scar formation. These include the Benelli mastopexy where a donut shaped piece of breast skin is excised from around the areola with an inner incision line following the perimeter of the areola and an outer incision line circling the areola further out. While this approach does minimize scar formation, it can result in serious stretching of the areola or tissue necrosis because all the newly lifted parenchymal weight of the breast is supported by suture surrounding the areola that is used to approximate the breast skin back to the circumference of the areola.

Several surgeons have attempted to reinforce their lift procedures using surgical meshes in open surgery mastopexy and breast reconstruction procedures. Some of these techniques have also incorporated the use of various reinforcing materials similar to those used in hernia repair, such as flat polymeric meshes, allografts, xenografts and autografts. However, wrapping materials around the parenchyma without the use of an anchoring element that shifts the load from the skin of the lower pole can leave the breast subject to the same ptotic forces that were present before breast surgery.

In 1981, Johnson described the use of MARLEX^{®} (polypropylene) mesh to convert the support of breast tissue after mastopexy from a cutaneous origin to a skeletal origin by attaching the mesh to the area of the second rib, (Johnson, Aesth. Plast. Surg. 5:77-84 (1981)). The flat MARLEX mesh is a permanent mesh made from polypropylene, and was implanted to provide two slings in each breast that supported the breast tissue. The procedure is based on the Wise open surgery breast lift.

Auclair and Mitz have described a mesh assisted mastopexy using a flat absorbable mesh and a periareolar skin resection technique (Auclair and Mitz, Ann. Chir. Plast. Esthét. 38:107-113 (1993)). A rapidly absorbing VICRYL^{®} mesh was placed around the anterior surface of the breast gland in order to form an internal bra. The procedure requires open surgery of the breast, and involves extensive dissection.

Góes has reported the use of polyglactin 910 (an absorbable copolymer of 90% glycolide and 10% L-lactide, also known as VICRYL) and a mixed mesh (containing 60% polyglactine 910 and 40% permanent polyester) in a periareolar mammoplasty using a double skin technique (Góes, Plast. Reconstr. Surg. 97:959-968 (1996)). The open surgery technique involves dissecting the soft tissue envelope away from the parenchyma, and wrapping the breast parenchyma with a mesh to help provoke the formation of a vigorous connective scar to produce a breast lining structure that would be less susceptible to ptosis.

US Patent No. 6,210,439 to Firmin et al. discloses a circular VICRYL mesh with a V-shaped opening extending from its center that has a metallic reinforcing wire running around the periphery. The implant is designed for insertion in an invasive open surgery procedure and assumes a conical shape suitable for mammoplasty when the reinforcing wire is tightened.

US Patent No. 7,476,249 to Frank discloses an implantable sling shaped prosthesis device for supporting and positioning a breast implant in a patient, wherein the device is configured from a sheet of a chemically inert permanent material, such as polytetrafluoroethylene or silicone, to support the breast implant. The sling shaped device is designed for placement in an open surgery invasive procedure.

US Patent Application Publication No. 2009/0082864 by Chen et al. also discloses a prosthetic device for supporting a breast implant made from a mesh. The device has a flat back wall, a concave front wall, and a curved transitional region between these walls that forms a smoothly curved bottom periphery. Insertion of the device requires an open surgery procedure with significant dissection.

US Patent Application No. 2008/0097601 by Codori-Hurff et al. discloses mastopexy and breast reconstruction procedures assisted by the use of processed tissue material derived from intestine or dermis. The tissue material is cut to a crescent shape to form an implant for mastopexy, and is implanted using an invasive open surgery procedure.

US Patent Application No. 20160038269 to Altman discloses various silk fabric implants that can be implanted using open surgery to support the lower breast.

US Patent Application No. 20120185041 to Mortarino et al. discloses knitted silk meshes that can be used to provide support to the lower pole of the breast.

US Patent Application No. 20130304098 to Mortarino discloses silk implants in the form of pockets that can be used in breast reconstruction.

WO 2009/001293 to Lauryssen discloses polypropylene and polyester mesh implants formed into cup shapes that can be used in mastopexy procedures. The meshes are implanted in open surgery procedures, and completely surround the breast tissue.

WO 2004/096098 to Hamilton discloses a permanent implant formed in a breast shape for soft tissue support, made from polytetrafluoroethylene (ePTFE), which can be used in forming a predetermined breast shape.

WO 2006/117622 to Lauryssen et al. discloses a permanent implant for soft tissue support of the breast that is generally L-shaped or U-shaped, and is wrapped around the breast to provide support.

Van Deventer et al. (Aesth. Plast. Surg. 36:578-89 (2012)) have disclosed the use of an internal breast support system for mastopexy using a partially degradable mesh that was formed into a cone (van Deventer et al. Aesth. Plast. Surg. 36:578-89 (2012)). The mesh is implanted in an open surgery procedure, and completely surrounds the upper and lower poles of the breast.

US Patent No. 9,532,867 to Felix discloses absorbable implants for breast surgery that conform to the breast parenchyma. The implants can support newly lifted breast parenchyma.

US20100023029 to Young discloses a sheet made from VICRYL for use in breast reconstruction with a number of attachment regions for attaching the sheet to the patient's anatomy. The device may be used to partially cover and constrain a tissue expander or implant.

WO2007004214 to Popov discloses a basket-shaped device for supporting the lower pole of a breast. The device is designed to be implanted using open surgery.

Several devices for performing open surgery mastopexy procedures by mimicking the breast's own fascial support system, the circum-mammary ligament, have been described.

US Patent Application No. 2017/0224471 to Rehnke discloses a circular tubular member with a purse string that can be used when the circum-mammary ligament has been stretched or weakened to lift the breast. The device is inserted in an open surgery procedure to tighten the circum-mammary ligament. The device is placed behind the breast gland on top of the pectoral muscle, anchored to the circum-mammary ligament, and cinched to a smaller diameter using the purse string. Cinching causes the base footprint of the breast to narrow, and gathers the breast together in a higher more projecting position on the patient's chest.

US Patent Application No. 2017/0231753 to Lee discloses a material that is placed on the pectoral muscle below the breast using open surgery to provide circumferential coverage of the breast tissue, stretching and securing the material above the breast, and attaching the material to the chest musculature medial and lateral to the breast in order to elevate the breast tissue.

Several devices for performing mastopexy procedures in a minimally invasive manner using slings have been described.

US Patent Application No. 2008/0027273 by Gutterman discloses a minimally invasive mastopexy system having a soft tissue support sling. The device is designed to provide support by suspending the breast from the upper pole region using a sling.

US Patent Application No. 2012/0283826 by Moses et al. discloses minimally invasive mastopexy systems having an insertion device, a suspension strut, and a lower pole support. The lower pole support is inserted beneath the lower pole of the breast as a sling to lift the breast.

US Patent Application No. 20100217388 to Cohen discloses cradling members for soft tissue shaping of the breast. The cradling member acts as a sling, and is implanted to lift the lower pole of the breast.

US Patent No. 7,670,372 to Shfaram et al. discloses a minimally invasive breast lifting system. The system incorporates one or more suspending members and a cradling member that is placed under the lower pole of the breast. The cradling member is connected to the suspending members, frequently, sutures, and acts as a sling to lift the breast.

US Patent Application Publication No. 2010/0331612 by Lashinski et al.*,* US2008/0082113 to Bishop et al.*,* and US 2009/0248071 to Saint et al. discloses a device for performing a minimally invasive mastopexy comprising a sling and soft tissue anchors that are introduced in a superior position to support the breast. US2012/0053689 to Martin et al. discloses PHA fiber for use in these devices.

US2010/137679A1 discloses a mastopexy system with anchor suspension lines secured to a superior sling.

Despite the advances described above, most mastopexy procedures require open surgical procedures with long surgical incisions that leave noticeable and permanent scars on the breast, particularly in the immediate period following the procedure. These scars can leave patients feeling dissatisfied with the aesthetic outcome of the procedure, and disappointed that the procedure failed to meet their expectations. Additionally, the procedures described generally involve extensive dissection of a tissue plane in the lower pole of the breast, and the insertion of a sling or other construct to lift or shape the lower pole. The extensive dissection in these existing procedures usually necessitates the use of general anesthesia.

It is an object and advantage of embodiments of the present invention to solve the shortcomings described above. For example, it is an object and advantage of embodiments of the present invention to provide implants and systems for mastopexy that can be used to provide a defined aesthetically pleasing outcome without open surgery, large incisions, and extensive dissection and manipulation of tissues, and that minimize the formation of permanent scars on the breast. Another object and advantage of the present invention is to provide implants and systems for mastopexy that can be used to avoid the use of scarring surgical incisions, dissection of tissue planes, the removal of excess skin, and the use of sutures to close incisions. Another object and advantage of the present invention is to provide mastopexy implants that can be implanted through small stab incisions. Such procedures could reduce operating time, and the need for general anesthesia. The latter would not only eliminate potential risks associated with general anesthesia, but would provide a more attractive and simpler mastopexy procedure, particularly for some younger patients that may prefer to delay more invasive procedures such as those described above. Another object and advantage of the present invention is to provide implants and systems for minimally invasive mastopexy that can be used in patients with fatty breasts, or less dense breast tissues, where lifting breast tissue is more challenging due to the higher content of fatty tissues present in such breasts.

### SUMMARY OF THE INVENTION

Implants and systems described herein assist the surgeon in reshaping, repositioning or lifting the breast to provide an aesthetically pleasing shape.

The implants and systems for mastopexy are designed to minimize the formation of scars. The implants may be implanted in the breast of a patient using blunt dissection, through small openings. The implants and systems for mastopexy avoid the use of traditional surgical approaches like the crescent mastopexy, donut (or Benelli) mastopexy, lollipop (or vertical) mastopexy, and the anchor (or Weiss or Wise) mastopexy, which all require extensive surgical incisions normally in conjunction with the removal of patient tissue for inserting mastopexy implants in the breast.

The implants and systems for mastopexy are designed to generate one or more new tissue planes in the breast. The present invention relates to mastopexy systems and methods for making such mastopexy systems as set out in the appended independent claims. Various embodiments are defined by the dependent claims.

### BRIEF DESCRIPTION OF THE DRAWINGS

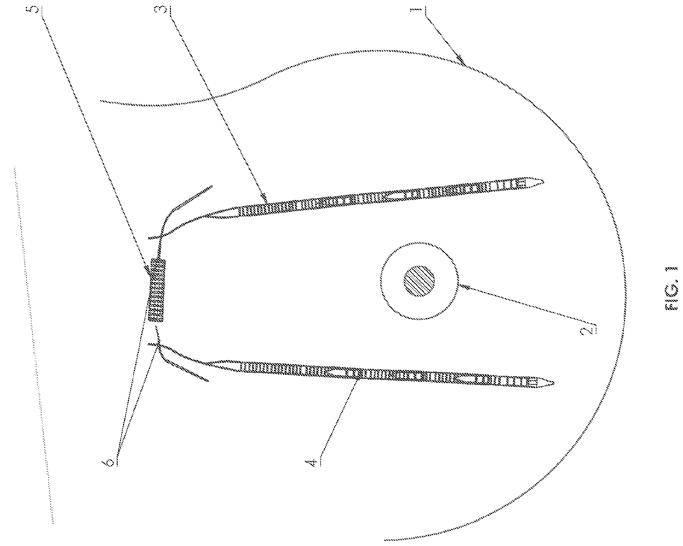

Figure 1 is a diagram showing the outline of a patient's breast 1 and a NAC 2, and implants for lifting the breast, namely, a first suspension member 3, a second suspension member 4, and a strut 5. After implantation and tensioning, the suspension members 3 and 4 are connected to the strut 5 at connection points 6.

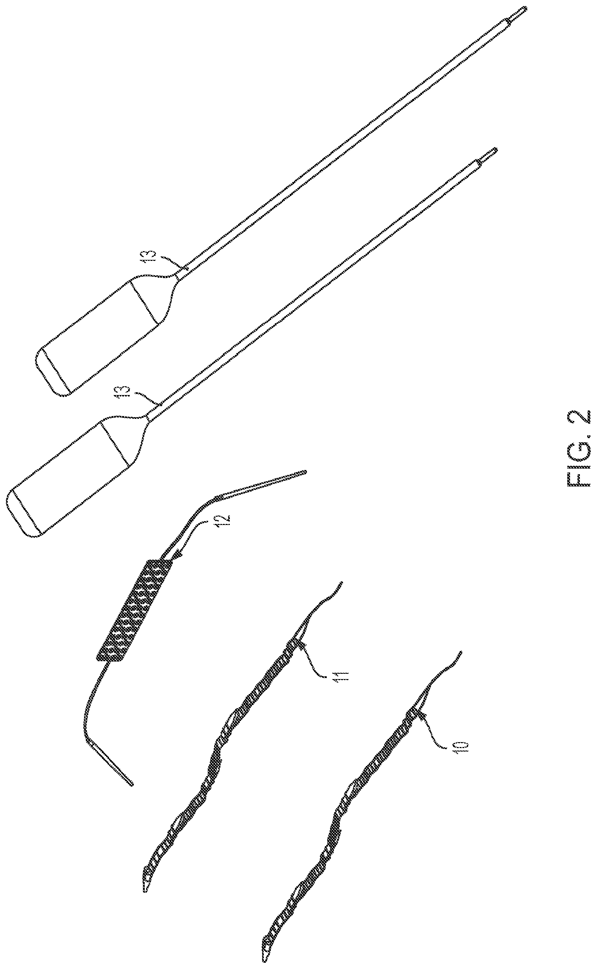

Figure 2 is a diagram showing an example of a system for mastopexy with a first suspension member 10, a second suspension member 11, a strut 12, and two introducer tools 13 with straight needles.

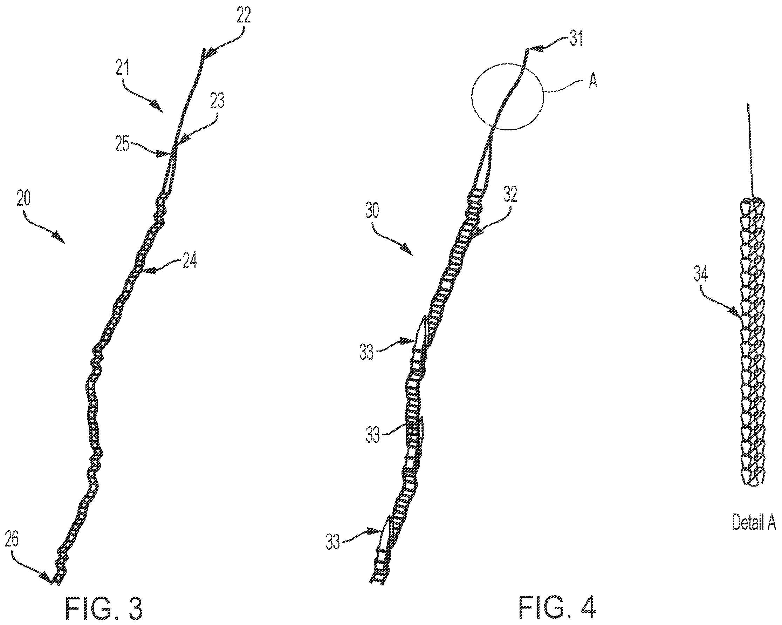

Figure 3 is a diagram of a suspension member 20 showing a support line 21 with a first end 22 and a second end 23, and an anchoring member 24 with a first end 25 and a second end 26, and the second end of the support line connected to the first end of the anchoring member.
Figure 4 is a diagram of a suspension member 30 with a support line 31 connected to an anchoring member with a ladder-shaped structure 32. The anchoring member is formed from three retainers 33 inserted in the ladder-shaped structure 32 . Detail A shows an expansion of area A of the support line 31, and shows fixation elements 34 formed with a two loop pillar stitch construction.

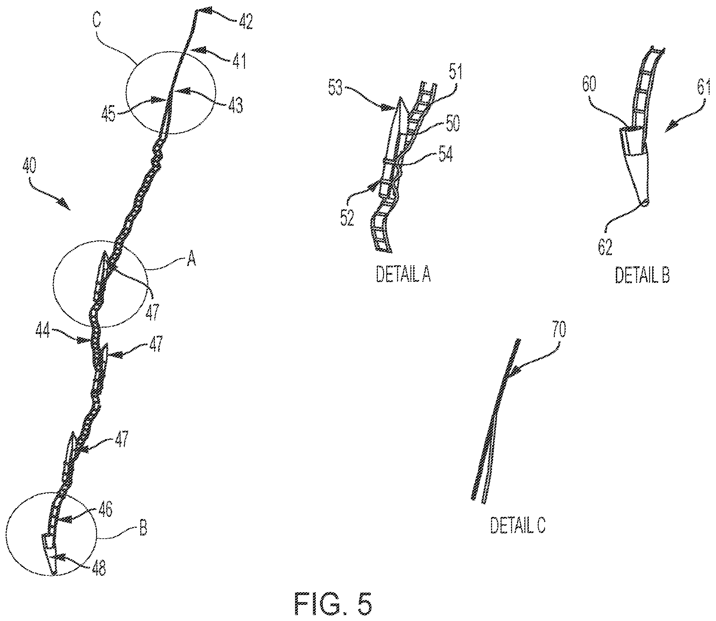

Figure 5 is a diagram of a suspension member 40 showing a support line 41 with a first end 42 and a second end 43, an anchoring member 44 with a first end 45, a second end 46, three retainers 47, and an introducer housing tip 48 connected to the second end of the anchoring member 46. Detail A is an expansion of area A, and shows a retainer 50, the ladder-shaped structure of the anchoring member 51, the retainer's base section 52, the retainer's tip 53, and the retainer secured in the crisscrossed ladder-shaped structure 54. Detail B is an expansion of area B, and shows the location 60 for inserting the introducer tool in the introducer housing tip 61, and the conical-shaped tip 62 used to channel the suspension member into the breast. Detail C is an expansion of area C, and shows the section of the support line 70 that may be connected to an implanted strut, fixated in tissue, or connected to the support line of a second suspension member.

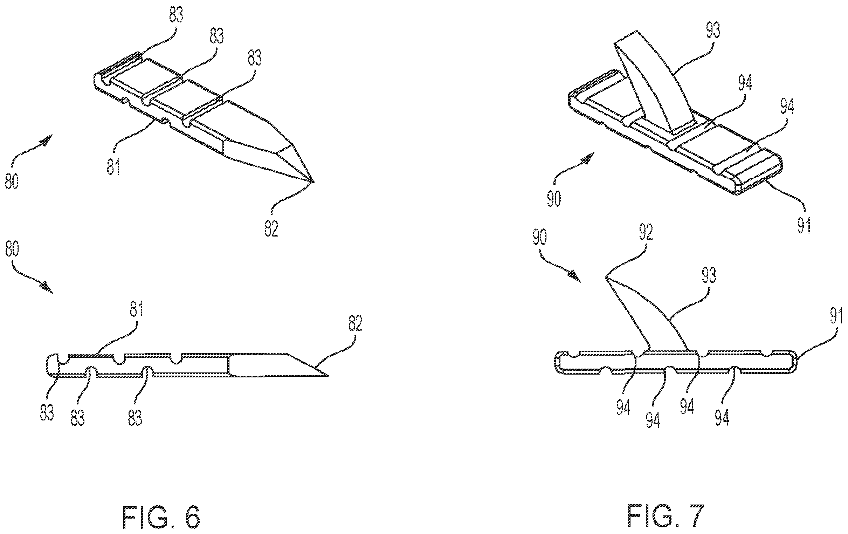

Figure 6 is a diagram showing two views of a retainer 80 with a base section 81, a tip 82, and grooves 83 for securing the retainer in a ladder-shaped structure of an anchoring member.
Figure 7 is a diagram showing two views of a retainer 90 with a base section 91, a tip 92, an angled tine 93, and grooves 94 for securing the retainer in a ladder-shaped structure of an anchoring member.

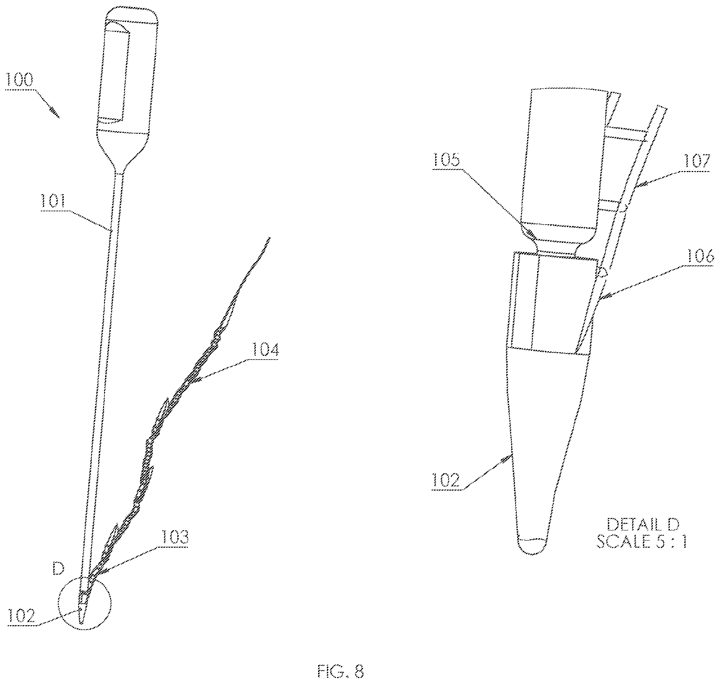

Figure 8 is a diagram showing an introducer tool 100 with a straight needle 101 inserted into an introducer housing tip 102 connected to an anchoring member 103 of a suspension member 104. Detail D is an expansion of area D, and shows the distal end of the introducer tool (105) inserted in the introducer housing tip 102, and the connection 106 of the introducer housing tip 102 to the ladder-shaped structure 107 of an anchoring member.

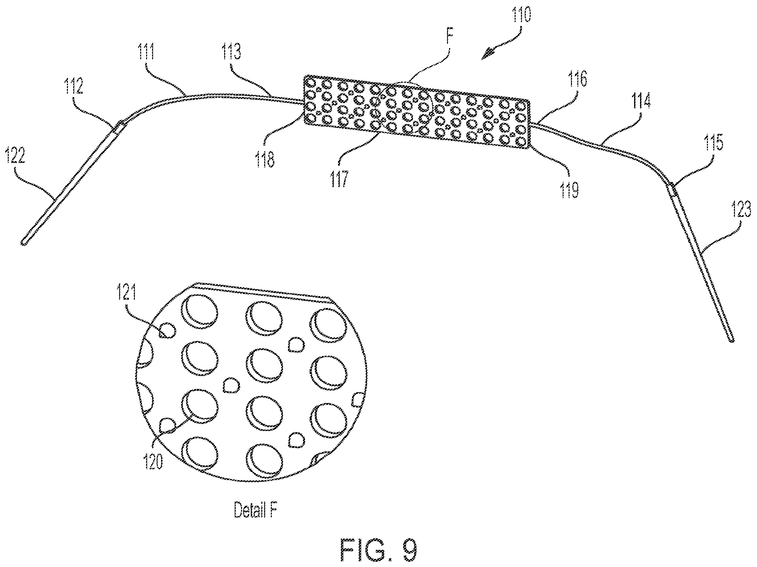

Figure 9 is a diagram showing a strut 110 with a first arm 111 having a first end 112 and a second end 113, a second arm 114 having a first end 115 and a second end 116, an element 117 with a first end 118 and a second end 119, with the second end of the first arm connected to the first end of the element, and the second end of the second arm connected to the second end of the element. A needle 122 with a blunt tip is connected to the first end of the first arm 111, and a second needle 123 with a blunt tip is connected to the first end 115 of the second arm 114. Detail F is an expansion of area F, and shows perforations 120 in the element, and tines 121 on the surface of the element.

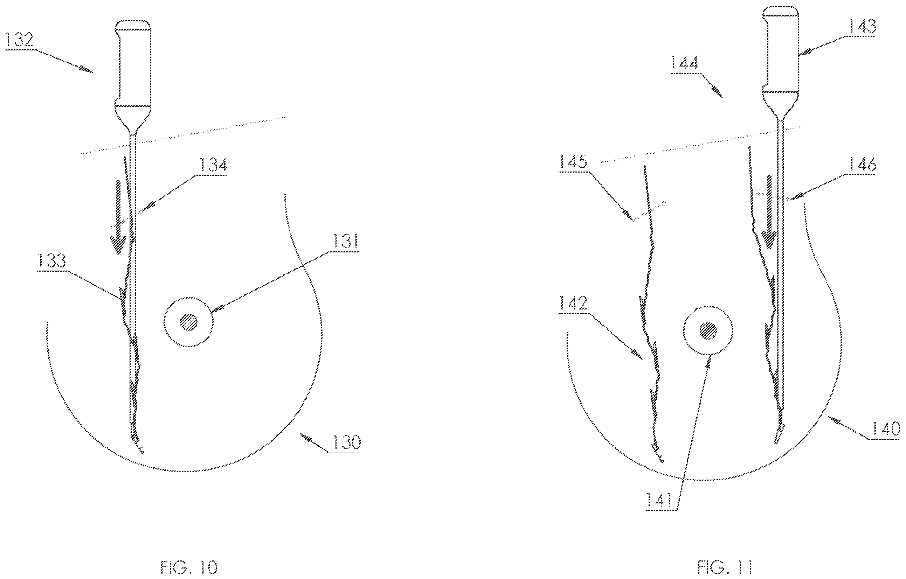

Figure 10 is a diagram showing the outline of a patient's breast 130 and the breast's NAC 131, and an introducer tool 132 loaded with a first suspension member 133 inserted on a first side of the patient's breast. The introducer tool loaded with the first suspension member is inserted in the breast in the direction indicated by the arrow, through stab incision location 134.
Figure 11 is a diagram showing the outline of a patient's breast 140 and the patient's NAC 141, a first suspension member 142 deployed on a first side of the patient's breast, and an introducer tool 143 loaded with a second suspension member 144 inserted on a second side of the patient's breast. The first suspension member is inserted in the patient's breast through stab incision location 145, and the second suspension member is inserted in the breast in the direction indicated by the arrow, through stab incision location 146.

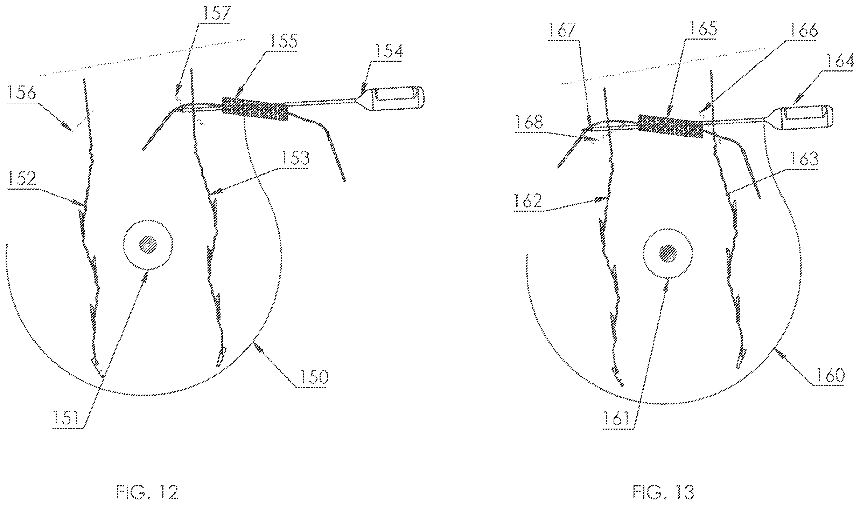

Figure 12 is a diagram showing the outline of a patient's breast 150 and the patient's NAC 151, a first suspension member 152, a second suspension member 153, and an introducer tool 154 loaded with a strut 155 for insertion of the strut in the patient's breast in a superior position to the patient's NAC. The first suspension member is inserted in the patient's breast through stab incision location 156, the second suspension member and the strut are inserted in the patient's breast through stab incision location 157.
Figure 13 is a diagram showing the outline of a patient's breast 160 and the patient's NAC 161, a first suspension member 162, a second suspension member 163, and an introducer tool 164 loaded with a strut 165 inserted through stab incision location 166, and the distal tip of the introducer tool 167 exiting through stab incision location 168. The first suspension member is inserted in the patient's breast through stab incision location 168, and the second suspension member is inserted in the patient's breast through stab incision location 166.

### DETAILED DESCRIPTION OF THE INVENTION

Before the present invention is described in detail, it is to be understood that this invention is not limited to particular variations set forth herein as various changes or modifications may be made to the invention described. As will be apparent to those of skill in the art upon reading this disclosure, each of the individual embodiments described and illustrated herein has discrete components and features which may be readily separated from or combined with the features of any of the other several embodiments

Methods recited herein may be carried out in any order of the recited events which is logically possible, as well as the recited order of events. Furthermore, where a range of values is provided, it is understood that every intervening value, between the upper and lower limit of that range and any other stated or intervening value in that stated range is encompassed within the invention.

Reference to a singular item, includes the possibility that there are plural of the same items present. More specifically, as used herein and in the appended claims, the singular forms "a," "an," "said" and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

Now turning to Figures 1-2, the anatomy of a patient's breast and a system for breast lift are depicted for facilitating understanding of the invention. Particularly, Fig. 1 is a diagram showing a patient's breast outline 1 and a NAC 2, and implants for lifting the breast composed of a first suspension member 3, a second suspension member 4 and a strut 5. The suspension members are inserted on the medial and lateral sides of the breast, and elevated in a superior direction to lift the breast. After tensioning of the suspension members to lift the breast, the suspension members may be secured directly to the patient's tissue, secured to each other, or preferably connected to a strut 5 that is inserted in the breast above the patient's NAC at connection points 6.

Figure 2 is diagram showing a system of implants and tools for mastopexy comprising a first suspension member 10, a second suspension member 11, a strut 12, and two straight needled introducer tools 13. The introducer tools are used to implant the first and second suspension members on the medial and lateral sides of the breast. The strut is implanted in the breast in a position superior to the NAC of the breast. After insertion in the breast, the first and second suspension members are lifted superiorly to tension the suspension members and lift the breast. The lifted breast may be held in place by connecting the tensioned suspension members to the strut, by securing the suspension members to the patient's tissue, or by connecting the suspension members to each other.

To further assist in understanding the following definitions are set forth below. However, it is also to be appreciated that unless defined otherwise as described herein, all technical and scientific terms used herein have the same meaning as commonly understood by one of ordinary skill in the art to which this invention belongs.

### DEFINITIONS

"Absorbable" as generally used herein means the material is degraded in the body, and the degradation products are eliminated or excreted from the body. The terms "absorbable", "resorbable", "degradable", and "erodible", with or without the prefix "bio", can be used interchangeably herein, to describe materials broken down and gradually absorbed, excreted, or eliminated by the body, whether degradation is due mainly to hydrolysis or mediated by metabolic processes.

"Bioactive agent" as generally used herein refers to therapeutic, prophylactic or diagnostic agents, preferably agents that promote healing and the regeneration of host tissue, and also therapeutic agents that prevent, inhibit or eliminate infection. "Bioactive agent" includes a single such agent and is also intended to include a plurality.

"Blend" as generally used herein means a physical combination of different polymers, as opposed to a copolymer formed of two or more different monomers.

"Burst strength" as generally used herein is determined according to ASTM D6797-02 (Standard Test Method for Bursting Strength of Fabrics Constant-Rate-of-Extension (CRE) Ball Burst Test) at ambient conditions using a ball burst fixture with a 1.6 cm circular opening and a 1cm diameter half-rounded probe.

"Copolymers of poly-4-hydroxybutyrate" as generally used herein means any polymer containing 4-hydroxybutyrate with one or more different hydroxy acid units.

"Endotoxin content" as generally used herein refers to the amount of endotoxin present in an implant or sample, and is determined by the limulus amebocyte lysate (LAL) assay.

"Inframammary fold" or "IMF" as generally used herein is the position where the lower pole of the breast meets the chest wall.

"Lower pole" as generally used herein means the part of the breast located between the inframammary fold (IMF) and the nipple meridian reference, and protruding away from the chest wall.

"Molecular weight" as generally used herein, unless otherwise specified, refers to the weight average molecular weight (Mw), not the number average molecular weight (Mn), and is measured by GPC relative to polystyrene.

"Poly-4-hydroxybutyrate" as generally used herein means a homopolymer containing 4-hydroxybutyrate units. It can be referred to herein as P4HB or TephaFLEX^{®} biomaterial (manufactured by Tepha, Inc., Lexington, MA).

"Retainer" as generally used herein means a shaped object that is incorporated into an anchoring member, and can engage tissue. The retainer generally has a sharp tip for engaging tissue. Examples of suitable retainers that can be incorporated into anchoring members include: hooks, darts, anchors, and barbs.

"Suture pullout strength" as generally used herein means the peak load (kg) at which an implant fails to retain a suture. It is determined using a tensile testing machine by securing an implant in a horizontal holding plate, threading a suture in a loop through the implant at a distance of 1 cm from the edge of the implant, and securing the suture arms in a fiber grip positioned above the implant. Testing is performed at a crosshead rate of 100 mm/min, and the peak load (kg) is recorded. The suture is selected so that the implant will fail before the suture fails.

"Upper pole" as generally used herein means the top part of the breast located between the upper pole reference and the nipple meridian reference, and protruding away from the chest wall.

### MATERIALS FOR PREPARING IMPLANTS FOR SCARLESS MASTOPEXY

In embodiments, implants and mastopexy systems have been developed using a wide variety of materials to perform mastopexy procedures by blunt dissection that cause minimal visible scar formation. The implants produce an aesthetically pleasing breast by lifting tissue in the breast. The implants create new tissue planes within the breast that can support the load of the lifted breast for a prolonged period. The implants avoid the need to use sling devices to lift the breast and extensive dissection of breast tissue for implantation of sling devices which can cause significant visible scarring of the breast.

### A. Polymers for Preparing Implants and Systems for Mastopexy

The mastopexy implants may comprise permanent materials, such as nondegradable thermoplastic polymers, including polymers and copolymers of ethylene and propylene, including ultra-high molecular weight polyethylene, ultra-high molecular weight polypropylene, nylon, polyesters such as poly(ethylene terephthalate), poly(tetrafluoroethylene), polyurethanes, poly(ether-urethanes), poly(methyl methacrylate), polyether ether ketone, polyolefins, and poly(ethylene oxide). However, the implants preferably comprise absorbable materials, more preferably thermoplastic or polymeric absorbable materials, and even more preferably the implants are made completely from absorbable materials. In embodiments, systems for mastopexy may comprise permanent materials used to facilitate delivery of the implant, and implants comprising absorbable materials. For example, the system for mastopexy may comprise an implant comprising a suspension member comprising an absorbable material, and a sheath used for delivery of the implant that is made from a permanent material.

In a preferred embodiment, the implants are made from one or more absorbable polymers, preferably absorbable thermoplastic polymers and copolymers. The implant may, for example, be prepared from polymers including, but not limited to, polymers of glycolic acid, lactic acid, 1,4-dioxanone, trimethylene carbonate, 1,4-butanediol, succinic acid, adipic acid, 3-hydroxybutyrate, 4-hydroxybutyrate, ε-caprolactone, including polyglycolic acid, polylactic acid, polydioxanone, polycaprolactone, copolymers of glycolic and lactic acids, such as VICRYL^{®}, MAXON^{®}, and MONOCRYL^{®} polymers, and including poly(lactide-co-caprolactones); poly(orthoesters); polyanhydrides; poly(phosphazenes); polyhydroxyalkanoates (PHA's); synthetically or biologically prepared polyesters; polycarbonates; tyrosine polycarbonates; polyamides (including synthetic and natural polyamides, polypeptides, and poly(amino acids)); polyesteramides; poly(alkylene alkylates); polyethers (such as polyethylene glycol, PEG, and polyethylene oxide, PEO); polyvinyl pyrrolidones or PVP; polyurethanes; polyetheresters; polyacetals; polycyanoacrylates; poly(oxyethylene)/poly(oxypropylene) copolymers; polyacetals, polyketals; polyphosphates; (phosphorous-containing) polymers; polyphosphoesters; polyalkylene oxalates; polyalkylene succinates; poly(maleic acids); silk (including recombinant silks and silk derivatives and analogs); chitin; chitosan; modified chitosan; keratin, biocompatible polysaccharides; hydrophilic or water soluble polymers, such as polyethylene glycol, (PEG) or polyvinyl pyrrolidone (PVP), with blocks of other biocompatible or biodegradable polymers, for example, poly(lactide), poly(lactide-co-glycolide), or polycaprolactone and copolymers thereof, including random copolymers and block copolymers thereof. Preferably the absorbable polymer or copolymer will be substantially resorbed after implantation within a 1 to 24-month timeframe, more preferably 3 to 18-month timeframe. Preferably, the absorbable polymer will retain some residual strength for at least 2 weeks to 3 months.

Blends of polymers, preferably absorbable polymers, can also be used to prepare the mastopexy implants. Particularly preferred blends of absorbable polymers are prepared from absorbable polymers including, but not limited to, polymers comprising glycolic acid, lactic acid, 1,4-dioxanone, trimethylene carbonate, 1,4-butanediol, succinic acid, adipic acid, 3-hydroxybutyrate, 4-hydroxybutyrate, ε-caprolactone or copolymers thereof.

In a particularly preferred embodiment, poly-4-hydroxybutyrate (Tepha's P4HB^{™} polymer, Lexington, MA) or a copolymer thereof is used to make the implant. Copolymers include P4HB with another hydroxyacid, such as 3-hydroxybutyrate, and P4HB with glycolic acid or lactic acid monomer. Poly-4-hydroxybutyrate is a strong, pliable thermoplastic polyester that is biocompatible and absorbable (Williams, et al. Poly-4-hydroxybutyrate (P4HB): a new generation of resorbable medical devices for tissue repair and regeneration, Biomed. Tech. 58(5):439-452 (2013)). Upon implantation, P4HB hydrolyzes to its monomer, and the monomer is metabolized via the Krebs cycle to carbon dioxide and water. In a preferred embodiment, the P4HB homopolymer and copolymers thereof have a weight average molecular weight, Mw, within the range of 50 kDa to 1,200 kDa (by GPC relative to polystyrene) and more preferably from 100 kDa to 600 kDa. A weight average molecular weight of the polymer of 50 kDa or higher is preferred for processing and mechanical properties.

In another preferred embodiment, the mastopexy implants comprise a polymer comprising at least a diol and a diacid. In a particularly preferred embodiment, the polymer used to prepare the mastopexy implant is poly(butylene succinate) (PBS) wherein the diol is 1,4-butanediol and the diacid is succinic acid. The poly(butylene succinate) polymer may be a copolymer with other diols, other diacids or a combination thereof. For example, the polymer may be a poly(butylene succinate) copolymer that further comprises one or more of the following: 1,3-propanediol, 2,3-butanediol, ethylene glycol, 1,5-pentanediol, glutaric acid, adipic acid, terephthalic acid, malonic acid, methylsuccinic acid, dimethylsuccinic acid, and oxalic acid. Examples of preferred poly(butylene succinate) copolymers are: poly(butylene succinate-co-adipate), poly(butylene succinate-co-terephthalate), poly(butylene succinate-co-butylene methylsuccinate), poly(butylene succinate-co-butylene dimethylsuccinate), poly(butylene succinate-co-ethylene succinate) and poly(butylene succinate-co-propylene succinate). The poly(butylene succinate) polymer or copolymer thereof may also further comprise one or more of the following: chain extender, coupling agent, cross-linking agent and branching agent. For example, the poly(butylene succinate) polymer or copolymer thereof may be branched, chain extended, or cross-linked by adding one or more of the following agents: malic acid, trimethylol propane, trimesic acid, citric acid, glycerol propoxylate, and tartaric acid. Particularly preferred agents for branching, chain extension, or crosslinking the poly(butylene succinate) polymer or copolymer thereof are hydroxycarboxylic acid units. Preferably the hydroxycarboxylic acid unit has two carboxylic groups and one hydroxyl group, two hydroxyl groups and one carboxyl group, three carboxyl groups and one hydroxyl group, or two hydroxyl groups and two carboxyl groups. In one preferred embodiment, the breast implants comprise poly(butylene succinate) comprising malic acid as a branching, chain extending, or cross-linking agent. This poly(butylene succinate) copolymer may be referred to as poly(butylene succinate) cross-linked or chain-extended with malic acid, succinic acid-1,4-butanediol-malic acid copolyester, or poly(1,4-butylene glycol-co-succinic acid), cross-linked or chain-extended with malic acid. It should be understood that references to malic acid and other cross-linking agents, coupling agents, branching agents and chain extenders include polymers prepared with these agents wherein the agent has undergone further reaction during processing. For example, the agent may undergo dehydration during polymerization. Thus, poly(butylene succinate)-malic acid copolymer refers to a copolymer prepared from succinic acid, 1,4-butanediol and malic acid. In another preferred embodiment, malic acid may be used as a branching, chain-extending or cross-linking agent to prepare a copolymer of poly(butylene succinate) with adipate, which may be referred to as poly[(butylene succinate)-co-adipate] cross-linked or chain-extended with malic acid. As used herein, "poly(butylene succinate) and copolymers" includes polymers and copolymers prepared with one or more of the following: chain extenders, coupling agents, cross-linking agents and branching agents. In a particularly preferred embodiment, the poly(butylene succinate) and copolymers thereof contain at least 70%, more preferably 80%, and even more preferably 90% by weight of succinic acid and 1,4-butanediol units. The polymers comprising diacid and diols, including poly(butylene succinate) and copolymers thereof and others described herein, preferably have a weight average molecular weight (Mw) of 10,000 Da to 400,000 Da, more preferably 50,000 Da to 300,000 Da and even more preferably 100,000 Da to 200,000 Da based on gel permeation chromatography (GPC) relative to polystyrene standards. In a particularly preferred embodiment, the polymers and copolymers have a weight average molecular weight of 50,000 Da to 300,000 Da, and more preferably 75,000 Da to 300,000 Da. In one preferred embodiment, the poly(butylene succinate) or copolymer thereof used to make the implant, or a component of the implant, has one or more, or all of the following properties: density of 1.23-1.26 g/cm³, glass transition temperature of -31 °C to -35 °C, melting point of 113 °C to 117 °C, melt flow rate (MFR) at 190 °C/2.16 kgf of 2 to 10 g/10 min, and tensile strength of 30 to 60 MPa.

### B. Additives

Certain additives may be incorporated into the implant, preferably in the absorbable polymer, copolymer or blends thereof that are used to make the implant. Preferably, these additives are incorporated during a compounding process to produce pellets that can be subsequently melt-processed. For example, pellets may be extruded into fibers suitable for making the implants. In another embodiment, the additives may be incorporated using a solution-based process, for example, fibers may be spun from solutions of the polymer and one or more additives.

In a preferred embodiment, the additives are biocompatible, and even more preferably the additives are both biocompatible and absorbable.

In one embodiment, the additives may be nucleating agents and/or plasticizers. These additives may be added in sufficient quantity to produce the desired result. In general, these additives may be added in amounts between 1% and 20% by weight. Nucleating agents may be incorporated to increase the rate of crystallization of the polymer, copolymer or blend. Such agents may be used, for example, to facilitate fabrication of the implant, and to improve the mechanical properties of the implant. Preferred nucleating agents include, but are not limited to, salts of organic acids such as calcium citrate, polymers or oligomers of PHA polymers and copolymers, high melting polymers such as PGA, talc, micronized mica, calcium carbonate, ammonium chloride, and aromatic amino acids such as tyrosine and phenylalanine.

Plasticizers that may be incorporated into the compositions for preparing the implants include, but are not limited to, di-n-butyl maleate, methyl laureate, dibutyl fumarate, di(2-ethylhexyl) (dioctyl) maleate, paraffin, dodecanol, olive oil, soybean oil, polytetramethylene glycols, methyl oleate, n-propyl oleate, tetrahydrofurfuryl oleate, epoxidized linseed oil, 2-ethyl hexyl epoxytallate, glycerol triacetate, methyl linoleate, dibutyl fumarate, methyl acetyl ricinoleate, acetyl tri(n-butyl) citrate, acetyl triethyl citrate, tri(n-butyl) citrate, triethyl citrate, bis(2-hydroxyethyl) dimerate, butyl ricinoleate, glyceryl tri-(acetyl ricinoleate), methyl ricinoleate, n-butyl acetyl rincinoleate, propylene glycol ricinoleate, diethyl succinate, diisobutyl adipate, dimethyl azelate, di(n-hexyl) azelate, tri-butyl phosphate, and mixtures thereof. Particularly preferred plasticizers are citrate esters.

### C. Bioactive Agents

The implants can be loaded or coated with bioactive agents. Bioactive agents may be included in the implants for a variety of reasons. For example, bioactive agents may be included in order to improve tissue in-growth into the implant, to improve tissue maturation, to provide for the delivery of an active agent, to improve wettability of the implant, to prevent infection, and to improve cell attachment. The bioactive agents may also be incorporated into the structure of the implant.

The implants may contain cellular adhesion factors, including cell adhesion polypeptides. As used herein, the term "cell adhesion polypeptides" refers to compounds having at least two amino acids per molecule that are capable of binding cells via cell surface molecules. The cell adhesion polypeptides include any of the proteins of the extracellular matrix which are known to play a role in cell adhesion, including fibronectin, vitronectin, laminin, elastin, fibrinogen, collagen types I, II, and V, as well as synthetic peptides with similar cell adhesion properties. The cell adhesion polypeptides also include peptides derived from any of the aforementioned proteins, including fragments or sequences containing the binding domains.

The implants can incorporate wetting agents designed to improve the wettability of the surfaces of the implant structures to allow fluids to be easily adsorbed onto the implant surfaces, and to promote cell attachment and or modify the water contact angle of the implant surface. Examples of wetting agents include polymers of ethylene oxide and propylene oxide, such as polyethylene oxide, polypropylene oxide, or copolymers of these, such as PLURONICS^{®}. Other suitable wetting agents include surfactants or emulsifiers.

The implants can contain gels, hydrogels or living hydrogel hybrids to further improve wetting properties and to promote cellular growth throughout the thickness of the scaffold. Hydrogel hybrids consist of living cells encapsulated in a biocompatible hydrogel like gelatin, methacrylated gelatin (GelMa), silk gels, and hyaluronic acid (HA) gels.

The implants can contain active agents designed to stimulate cell in-growth, including growth factors, cellular differentiating factors, cellular recruiting factors, cell receptors, cell-binding factors, cell signaling molecules, such as cytokines, and molecules to promote cell migration, cell division, cell proliferation and extracellular matrix deposition. Such active agents include fibroblast growth factor (FGF), transforming growth factor (TGF), platelet derived growth factor (PDGF), epidermal growth factor (EGF), granulocyte-macrophage colony stimulation factor (GMCSF), vascular endothelial growth factor (VEGF), insulin-like growth factor (IGF), hepatocyte growth factor (HGF), interleukin-1-B (IL-1 B), interleukin-8 (IL-8), and nerve growth factor (NGF), and combinations thereof.

Other bioactive agents that can be incorporated in the implants include antimicrobial agents, in particular antibiotics, disinfectants, oncological agents, anti-scarring agents, anti-inflammatory agents, anesthetics, small molecule drugs, anti-angiogenic factors and pro-angiogenic factors, immunomodulatory agents, and blood clotting agents. The bioactive agents may be proteins such as collagen and antibodies, peptides, polysaccharides such as chitosan, alginate, keratin, hyaluronic acid and derivatives thereof, nucleic acid molecules, small molecular weight compounds such as steroids, inorganic materials such as hydroxyapatite, or complex mixtures such as platelet rich plasma. Suitable antimicrobial agents include: bacitracin, biguanide, triclosan, gentamicin, minocycline, rifampin, vancomycin, cephalosporins, copper, zinc, silver, and gold. Nucleic acid molecules may include DNA, RNA, siRNA, miRNA, antisense or aptamers.

The implants may also contain allograft material and xenograft materials, including acellular dermal matrix material and small intestinal submucosa (SIS).

Additionally, human fat such as autologous fat grafts may be added or injected across or into the implant scaffolding. Lipoaspirate fatty tissue from the patient may be added to the internal surface or external surface of the implant. In areas where the implant is porous, the fatty tissue and globules may be held in place within the pores of the implant.

In another embodiment, the collected fatty tissue is mixed with a natural or synthetic fluidized scaffolding matrix to be added to the implant to assist in holding the globules of fat in place in the implant. Examples of natural and synthetic fluidized scaffolding matrix include, without limitation, hydrogels, water soluble polymers, polyesters, and hydrophilic polymers, including polyethylene oxide, polyvinyl alcohol, and polymers of fibrin, thrombin, alginate, collagen, chitosan, and silk.

In yet another preferred embodiment, the implants may incorporate systems for the controlled release of the therapeutic or prophylactic agents.

### COMPONENTS FOR PREPARING MASTOPEXY IMPLANTS

A variety of methods can be used to manufacture the implants and systems for mastopexy. The implants may comprise the fibers disclosed herein.

### Fibers for Making Mastopexy Implants

The implants may comprise fibers. The fibers are preferably made from absorbable thermoplastic polymers, and even more preferably from absorbable thermoplastic polyesters. The fibers are preferably made from the absorbable materials listed above. The fibers may be monofilament fibers, multifilament fibers, or combinations thereof. Particularly preferred implants comprise monofilament fibers. The fibers may be unoriented, partially oriented, highly oriented or combinations thereof, but are preferably oriented. The fibers preferably have elongation to break values of 3% to 100%, more preferably 3% to 50%. The fibers may have diameters ranging from 1 micron to 5 mm, more preferably from 10 microns to 1 mm, and even more preferably from 50 microns to 500 microns. The fibers may have weight average molecular weights ranging from 10 kDa to 1,200 kDa, but more preferably from 50 kDa to 600 kDa. The fibers preferably retain at least 50% of their initial strength *in vivo* for 1-6 months, more preferably for 2-4 months. The fibers preferably completely degrade within 5 years of implantation, and more preferably within 2 years of implantation. The fibers preferably have initial tensile strengths ranging from 1 to 1,300 MPa, and more preferably from 50 MPa to 1,000 MPa.

In an embodiment, the implants comprise fibers with one or more of the following properties: an elongation to break of 10-100%, and a tensile strength of 300-1,000 MPa.

In one preferred embodiment, the mastopexy implants comprise fibers made from P4HB, and more preferably from P4HB monofilament fiber. The P4HB monofilament fibers are preferably partially or fully oriented (i.e. partially or fully stretched after extrusion). In one embodiment, P4HB monofilament fiber may be produced according to the following method. Bulk P4HB resin in pellet form is dried to under 300 ppm water using a rotary vane vacuum pump system. The dried resin is transferred to an extruder feed hopper with nitrogen purge to keep the pellets dry. The pellets are gravity fed into a chilled feeder section and introduced into an extruder barrel, with a 1.5 inch (3.8 cm) diameter, and fitted with an extrusion screw with a 30:1 L/D ratio. The extruder barrel preferably contains 5 heating zones (or extrusion zones), and is manufactured by American Kuhne. The heated and softened resin from the extruder is fed into a heated metering pump (melt pump) and from the melt pump the extruded resin is fed into the heated block and an 8-hole spinneret assembly. Suitable processing profile ranges are from 40°C to 260°C for temperatures, and 400 psi to 2000 psi (2.76 MPa to 13.8 MPa) for pressures. The molten filaments are preferably water quenched and optionally conveyed into an orientation line, preferably a three-stage orientation line, and optionally with inline relaxation, before winding of the monofilaments on spools. This procedure may, for example, be used to produce P4HB monofilament fibers with one or more of the following properties: an elongation to break from 10-100%, a tensile strength from 50-1,300 MPa, and a tensile modulus from 70-1,000 MPa. The P4HB monofilament fibers may have average diameters ranging from 20 microns to 1 mm, but are more preferably 50 microns to 500 microns. In an embodiment, the P4HB monofilament fibers may have USP (United States Pharmacopeia) sizes 10, 9, 8, 7, 6, 5, 4, 3, 2, 1, 0, 2-0, 3-0, 4-0, 5-0, 6-0, 7-0, 8-0, 9-0, 10-0, 11-0, and 12-0.

In another embodiment, the mastopexy implants comprise fibers made from P4HB multifilament fiber. Multifilament fibers of P4HB or copolymers thereof may be spun, for example, as follows: The polymer, copolymer or blend thereof is pelletized, and dried so that the moisture content of the polymer, copolymer or blend is less than 300 ppm. The dried pellets are placed in the feed hopper of an extruder, and protected from moisture, for example with a dry nitrogen purge. The pellets are gravity fed into a chilled feeder section, and introduced into a suitable extruder barrel with an extrusion screw. One suitable extruder barrel has a diameter of 0.75 inches (1.91 cm) and length of 25.69 inches (65.3 cm), and is fitted with an extrusion screw with a 30:1 L/D ratio. American Kuhne makes a suitable extruder. In a preferred embodiment, the extruder barrel contains 4 heating zones, and a processing profile is set with temperatures ranging from 40 °C to 300°C and pressures of 200 psi to 3,000 psi (1.38 MPa to 20.7 MPa). The heated and softened polymer, copolymer or blend is fed into a metering pump, and from the metering pump the resin is fed into the heated block. The spin head is fitted with a spin pack comprising filtering media (screens), and spinnerets containing the desired number of holes for forming the individual filaments of the multifilament yarn. For example, the spinneret may have 15, 30, 60, 120 or more or less holes. The extruded filaments exit the spinneret, and pass through a heated chimney before they are allowed to cool. Spin finish is preferably applied to the yarn, and the yarn may either be collected on a winder, or oriented in-line. Suitable spin finishes include PEG400 and Tween 20^{™}. The multifilament fiber may have a tenacity between 1 and 12 grams per denier.

In another preferred embodiment, the mastopexy implants comprise fibers made from poly(butylene succinate) or copolymer thereof, and more preferably from monofilament fibers of poly(butylene succinate) or copolymer thereof. The monofilament fibers of poly(butylene succinate) or copolymer thereof are preferably partially or fully oriented (i.e. partially or fully stretched after extrusion). In one embodiment, monofilament fibers of poly(butylene succinate) or copolymer thereof may be produced according to the following method. Bulk resin is dried under vacuum overnight to less than 0.01% (w/w) water. Dried pellets of the polymer are fed under a blanket of nitrogen into the extruder barrel of a 2 ½" American Kuhne single screw extruder (30:1 L:D, 3:1 compression) equipped with a Zenith type metering pump model HPB917, a die with 0.5 mm - 8 hole spinneret and 8 heat zones. The 8 heating zones of the extruder are set between 40°C and 200°C. The extruder is fitted with a quench bath filled with water at 35 - 70°C and set up with an air gap of 10 mm between the bottom of the spinneret and the surface of the water. Two 5-roll godets are positioned after the quench bath, followed by three sets of hot conduction chambers fed by godets in order to orient the fiber in multiple stages. The temperatures of the hot chambers are set between 50° to 90°C temperature. Another godet is positioned after the last chamber, and then a multi-position Sahm winder. This procedure may, for example, be used to produce monofilament fibers of poly(butylene succinate) or copolymer thereof with one or more of the following properties: an elongation to break from 10-100%, or more preferably 10-50%, a tensile strength from 50-1,300 MPa, or more preferably 400-1,200 MPa, and a tensile modulus from 50-3,000 MPa. The monofilament fibers of poly(butylene succinate) and copolymers thereof may have average diameters ranging from 20 microns to 1 mm, but are more preferably 50 microns to 500 microns. In an embodiment, the monofilament fibers of poly(butylene succinate) or copolymers thereof may have USP (United States Pharmacopeia) sizes 10, 9, 8, 7, 6, 5, 4, 3, 2, 1, 0, 2-0, 3-0, 4-0, 5-0, 6-0, 7-0, 8-0, 9-0, 10-0, 11-0, and 12-0.

In another embodiment, the mastopexy implants comprise multifilament fiber made from poly(butylene succinate) and copolymers thereof. The multifilament fibers of poly(butylene succinate) or copolymer thereof are preferably partially or fully oriented (i.e. partially or fully stretched after extrusion). In one embodiment, the multifilament fibers of poly(butylene succinate) or copolymer thereof may be produced according to the following method. Bulk resin is dried under vacuum to less than 0.01% (w/w) water. Dried pellets of the polymer are fed into an extruder barrel of an AJA (Alex James Associates, Greer, S.C.) ¾" single screw extruder (24:1 L:D). The extrusion barrel contained 4 heating zones, a metering pump and a spin pack assembly. The pellets are gravity fed into a chilled feeder section and introduced into the extruder with temperature profile set as follows: Chimney 40°C - 100°C, Spinneret 170°C ± 30°C, Pump 170°C ± 30°C, Block 170°C ± 30°C, Zone 4 160°C ± 40°C, Zone 3 150°C ± 40°C, Zone 2 120°C ± 50°C, Zone 1 30°C - 40°C, Feed Zone: Ambient temperature. The heated and homogenized melted resin from the extruder is fed into a heated metering pump (melt pump), and from the melt pump the extruded resin is fed into the heated block and the spinneret assembly. The spinneret has 30 holes with a capillary diameter of 0.200 millimeters and a L/D ratio of 2:1. (The spinneret may also be configured in other alternative manners. For example, the spinneret can be configured with capillary diameters from 0.150 to 0.300 millimeters (6 mil to 12 mil) and 15, 120 and 240 holes, as well as higher and lower diameters and numbers of holes.) Suitable processing temperature profile ranges are from 35°C to 250°C with pressures ranging from 200 to 5,000 psi (1.38 MPa to 34.5 MPa) in the barrel and 200 to 5,000 psi (1.38 MPa to 34.5 MPa) in the spin pack. As the molten filaments exit the spin pack they pass through a heated chimney collar that is 6 - 12 inches long and range in temperature from 40°C to 100°C, and then through an air quench box. The spin pack is suspended vertically above a yarn take-up roll at a distance sufficient to allow crystallization of the molten filaments and application of spin finish lubricant. A spin finish solution of 25% polyethylene 25 glycol 400 (PEG400) in water is used to hold the filaments together to form a yarn bundle. The speed of the yarn take-up rolls (typically 3-18 meters per minute) is set in proportion to the flow rate of the molten filament to control the denier of the as spun yarn bundle. The as spun yarn bundle is then conveyed to a Lessona winder for offline later orientation or conveyed to a take-up roll for inline orientation on a series of cold and heated godet pairs and separator rolls. If desired, the spin finish can be reactivated by rewetting the yarn bundle with pure water, and the yarn drawn at ratios from 5 to 14X and temperatures ranging from 50°C to 90°C. This procedure may, for example, be used to produce multifilament fibers of poly(butylene succinate) or copolymer thereof with one or more of the following properties: an elongation to break from 10-100%, or more preferably 10-50%, and a tenacity greater than 1 gram per denier, more preferably greater than 4 grams per denier, but less than 14 grams per denier.

### Textiles for Making Mastopexy Implants

The fibers described herein may be processed into textiles, for example, by knitting, stitching, weaving, or crocheting. A particularly preferred textiles for preparing the mastopexy implants is a two loop pillar construction. Another particularly preferred textile for use in preparing the mastopexy implants is a mesh, or a warp knit mesh, and more preferably an absorbable warp knit mesh.

In embodiments, textiles and meshes used to prepare the mastopexy implants have burst strengths between 0.6 and 90 N/cm², more preferably between 1.2 and 30 N/cm². In embodiments, textiles and meshes used to prepare the mastopexy implants have burst strengths 3 months after implantation of at least 40% of their initial burst strength values.

In embodiments, textiles and meshes used to prepare the mastopexy implants are porous, and can be replaced *in vivo* by host tissue growing into and around the implant that is strong enough to support the breast. The diameters of the mesh pores or pores in the textile are preferably larger than 25 µm, more preferably larger than 75 µm, and even more preferably larger than 250 µm in order to facilitate tissue in-growth, but smaller than 20 mm, more preferably smaller than 10 mm, and even more preferably smaller than 5 mm.

Mastopexy implants comprising textiles, such as knitted and woven meshes, and two loop pillar construction textiles, may be produced using fibers of P4HB or copolymer thereof, and fibers of poly(butylene succinate) or copolymer thereof. Preferably the fibers are monofilament fibers. Implants comprising oriented or partially oriented monofilament fibers of these polymers and copolymers have a prolonged strength retention profile, and can maintain some residual strength for as much as one year. The prolonged strength retention of these fibers provides an extended period for tissue in-growth into textiles made from these fibers. Tissue in-growth into these textiles leads to the formation of a new tissue plane in the breast that can support a breast lift. Moreover, the prolonged strength retention of the fibers and textiles means that mastopexy implants comprising these fibers and textiles can provide short-term support for the lifted breast while the new tissue plane develops in the breast.

In other embodiments, mastopexy implants comprising textiles, including two loop pillar constructs, or knitted meshes may be produced using fibers of polydioxanone, preferably monofilament fibers of polydioxanone, and even more preferably oriented monofilament fibers of polydioxanone.

A suitable knitted P4HB mesh for use in the mastopexy implants may be prepared, for example, by the following method. Monofilament fibers of P4HB from 49 spools are pulled under uniform tension to the surface of a warp beam. A warp is a large wide spool onto which individual fibers are wound in parallel to provide a sheet of fibers ready for coating with a 10% solution of Tween^{®} 20 lubricant. Tween 20 lubricant is added to the surface of the sheet of fiber by means of a 'kiss' roller that is spinning and is immersed in a bath filled with Tween 20. The upper surface of the roller is brought into contact with the sheet of fiber, and the roller spun at a uniform speed to provide a consistent application of Tween 20 finish. Following the application of Tween 20, the sheet of fiber is placed onto a creel position such that each spooled fiber is aligned and wrapped side by side to the next spooled fiber on a warp beam. Next, warp beams are converted into a finished mesh fabric by means of interlocking knit loops. Eight warp beams are mounted in parallel onto a tricot machine let-offs and fed into the knitting elements at a constant rate determined by the 'runner length'. Each individual monofilament fiber from each beam is fed through a series of dynamic tension elements down into the knitting 'guides'. Each fiber is passed through a single guide, which is fixed to a guide bar. The guide bar directs the fibers around the needles forming the mesh structure. The mesh fabric is then pulled off the needles by the take down rollers at a constant rate of speed. The mesh fabric is then taken up and wound onto a roll. The P4HB monofilament mesh produced according to this method may be scored ultrasonically with water, optionally heat set in hot water, and optionally washed with a 70% aqueous ethanol solution.

In embodiments, textiles and meshes made from P4HB monofilaments have one or more of the following properties: (i) a suture pullout strength of at least 1 Kgf, (ii) a burst strength of 0.1 to 100 Kg, (iii) a thickness of 0.05-5 mm, (iv) an areal density of 5 to 800 g/m², and (v) a pore diameter of 5 µm to 5 mm. In embodiments, the textiles and monofilament meshes have one or more of the following properties: (i) a suture pullout strength of 1 Kgf to 20 Kgf, (ii) a burst strength of 1 to 50 Kg, more preferably 10 to 50 Kg, (iii) a thickness of 0.1 to 1 mm, (iv) an areal density of 100 to 300 g/m², and (v) a pore diameter 100 µm to 1 mm. In embodiments, the P4HB monofilament mesh or textile has substantially one or more of the following properties: a pore diameter of 500±100 µm, a thickness of 0.5±0.2 mm, an areal density of approx. 182±50 g/m², a suture pullout strength of 5.6±2 kgf, and a burst strength of at least 15 Kg, and more preferably at least 24.5 Kg.

A suitable knitted mesh of poly(butylene succinate) or copolymer thereof for use in the mastopexy implants may be prepared, for example, by the following method. Monofilament fibers from 49 spools are mounted on a creel, aligned side by side and pulled under uniform tension to the upper surface of a "kiss" roller. The "kiss" roller is spinning while semi-immersed in a bath filled with a 10% solution of polyethylene glycol sorbitan monolaurate, polyethylene glycol, or other suitable lubricant. The lubricant is deposited on the surface of the sheet of fiber. Following the application of the lubricant, the sheet of fiber is passed into a comb guide and then wound on a warp beam. A warp is a large wide cylinder onto which individual fibers are wound in parallel to provide a sheet of fibers. Next, warp beams are converted into a finished mesh fabric by means of interlocking knit loops. Eight warp beams are mounted in parallel onto tricot machine let-offs and fed into the knitting elements at a constant rate determined by the 'runner length'. Each individual monofilament fiber from each beam is fed through a series of dynamic tension elements down into the knitting 'guides'. Each fiber is passed through a single guide, which is fixed to a guide bar. The guide bar directs the fibers around the needles forming the mesh fabric structure. The mesh fabric is then pulled off the needles by the take down rollers at a constant rate of speed determined by the fabric 'quality'. The mesh fabric is then taken up and wound onto a roll ready for scoring. The poly(butylene succinate) or copolymer thereof monofilament mesh is then scoured ultrasonically with water, and may be (i) heat set (for example in a hot conductive liquid bath or an oven), and then (ii) washed with a 70% aqueous ethanol solution.

Meshes and textiles, including a two loop pillar construct, made from monofilaments or multifilaments of poly(butylene succinate) or copolymers thereof preferably have one or more of the following properties: (i) a suture pullout strength of at least 10 N, or at least 20 N, (ii) a burst strength of 0.1 to 100 kgf, more preferably between 1 to 50 kgf, or greater than 0.1 kPa, (iii) a thickness of 0.05-5 mm, (iv) an areal density of 5 to 800 g/m², (v) a pore diameter of 5 µm to 5 mm, or more preferably 100 µm to 1 mm, and (vi) Taber stiffness of at least 0.01 Taber Stiffness units, more preferably 0.1-19 Taber Stiffness units. More preferably, these monofilament or multifilament meshes and textiles have one or more of the following properties: (i) a suture pullout strength of 1 kgf to 20 kgf, (ii) a burst strength of 1 to 50 kgf, more preferably 5 to 30 kgf, (iii) a thickness of 0.1 to 1 mm, (iv) an areal density of 100 to 300 g/m², and (v) a pore diameter of 100 µm to 1 mm. In another preferred embodiment, the monofilament or multifilament mesh or textile of poly(butylene succinate) or copolymer thereof has substantially one or more of the following properties: a pore diameter of 500±100 µm, thickness of 0.4±0.3 mm, areal density of approx. 182±50 g/m², suture pullout strength of 5.6±2 kgf, and a burst strength of at least 3 kgf, and more preferably at least 6 kgf.

### METHODS FOR PREPARING MASTOPEXY IMPLANTS AND SYSTEMS FOR MASTOPEXY

A variety of methods can be used to manufacture the mastopexy implants and systems for mastopexy. Preferably, the mastopexy implants are absorbable, and are designed to support the mechanical forces acting on the breast during normal activities at the time of implantation, and to allow a steady transition of mechanical forces to regenerated host tissues that can also support those same mechanical forces once the implant has degraded.

### Suspension Members

In embodiments, the mastopexy implants comprise suspension members. The suspension members can be used to lift the breast of a patient. The suspension members are not slings that are designed to make a hammock underneath the lower pole of the breast in order to lift the breast. Instead, one or more suspension members are implanted on the lateral side of the breast to lift the lateral side of the breast, and one or more suspension members are implanted on the medial side of the breast to lift the medial side of the breast. In a preferred embodiment, suspension members are implanted on both sides of the breast in order to lift the breast. In embodiments, a suspension member 20 comprises a support line 21 and an anchoring member 24 (see Fig. 3). The support line 21 has a first end 22 and a second end 23. The anchoring member 24 has a first end 25 and a second end 26. The suspension members comprise a support line connected to an anchoring member. The support line 21 is connected at its second end 23 to the anchoring member 24 at its first end 25. The anchoring member 24 is only connected to a support line 21 at its first end 25. The anchoring member 24 is not connected to a second support line at its second end 26. In a sense, the second end of the anchoring member is shown as a free end in FIG. 3.

The suspension members 20 are designed to lift the breast of a patient by engaging breast tissue with the anchoring members 24, and lifting the engaged tissue by applying a force on the support lines 21 in an inferior to superior direction. The upward force on the support lines is transmitted to the anchoring members, and results in upward movement of the breast tissue. In embodiments, the suspension members are designed to be implanted on the lateral and medial sides of the breast in order to lift the breast.

The suspension members are designed to be transitory scaffolds. That is, the suspension members are designed to initially lift the breast, but be replaced by new tissue after implantation as the suspension member degrades. Over time, the support of the breast provided by the suspension member is replaced by support from host tissue. In this manner, the breast is lifted by the suspension member, but maintained longer-term in its lifted position by in-grown breast tissue. In embodiments, the suspension members retain at least 15% of their initial strength, preferably at least 30% of their initial strength, and even more preferably at least 50% of their initial strength for 12 weeks following implantation.

In embodiments, the suspension members are designed to create new tissue planes in the breast that can be used to support a lifted breast as the suspension members degrade and lose strength. In embodiments, the suspension members are designed to distribute the load of the lifted breast over the area of the new tissue plane. Use of the suspension members to distribute the load of the breast provides a more durable result, and avoids concentration of the load, potential cheese wiring, and loss of tissue traction that can occur when only sutures are used in breast lift procedures. In this regard, the suspension members may be particularly desirable in lifting breasts with high fatty tissue contents where sutures tend to pull through the breast tissue. We have discovered that the width/profile of the suspension members 20 does not need to be large in order to spread the load of the lifted breast over an extended area and still be effective. The low profile suspension member designs described herein (a) allow the suspension member to be deployed through only a stab wound and narrow channel; (b) can adequately support the breast; and (c) avoid the above mentioned problems associated with sutures and slings.

In order to lift the breasts, the suspension members are designed to support a load of at least 5 N, more preferably at least 10 N, and more preferably at least 60 N, but less than 500 N.

In embodiments, the suspension members comprise a porous component. The porous component permits tissue in-growth.

The suspension members may further comprise a tensioner. The tensioner is designed to increase or decrease the tension of the suspension member on the breast tissue, and allow adjustment of the breast lift.

The suspension members are sized for implantation through stab incisions made in the breast of the patient. In embodiments, the suspension members are designed for use without extensive dissection of the patient's tissue. In embodiments, the suspension members are designed for use without removal of the patient's tissue. In embodiments, the suspension members are designed for minimally invasive delivery. In embodiments, the suspension members are designed to be folded or rolled up prior to implantation in the patient. After implantation, the folded or rolled up suspension members may be deployed in the breast into their desired shapes. In embodiments, the suspension members have shape memory. After implantation, the suspension members with shape memory may be deployed in the breast into their desired shapes. In embodiments, the suspension members are designed to be implanted in channels formed in the breast preferably by blunt dissection. In embodiments, the suspension members are designed to be implanted through entry points in the upper pole of the breast. In embodiments, the suspension members are designed to be implanted through entry points in the lower pole of the breast.

In embodiments, the suspension members comprise fibers. In embodiments, the fibers are made from absorbable thermoplastic polymers, and in embodiments the fibers made from absorbable thermoplastic polymers are oriented. Oriented means that the fibers have been stretched. Stretching of the fiber causes molecular alignment of the polymer chains in the fiber, and increases the tensile strength of the fiber. ("Oriented" in this context does not relate to the geometry of a fiber in relation to another object.) In embodiments, the suspension members comprise monofilament fibers, preferably absorbable monofilament fibers, and even more preferably oriented polymeric absorbable monofilament fibers.

In embodiments, the suspension members comprise one or more of the polymers listed in Section A. Preferably, the suspension members comprise one or more of the following: poly-4-hydroxybutyrate or copolymer thereof, poly(butylene succinate) or copolymer thereof, or polydioxanone.

In embodiments, the implant comprises a suspension member and one or more bioactive agents. Bioactive agents may be coated on the suspension members, or incorporated into the suspension members. Particularly preferred bioactive agents include anti-microbials, antibiotics, proteins, and agents to promote tissue in-growth. In embodiments, the implant comprises a suspension member and one or more of the following: cells, stem cells, gels, hydrogels, fatty tissue, autologous fat, fat lipoaspirate, adipose cells, and fibroblast cells. These cells and materials may be coated onto the surfaces of the suspension members or incorporated into the body of the suspension members.

In embodiments, the suspension member has an endotoxin content of less than 20 endotoxin units. In embodiments, the suspension member is sterile. In embodiments, the suspension member is sterilized with ethylene oxide, cold ethylene oxide, electron-beam irradiation, or gamma-irradiation.

### Support Lines

In embodiments, the suspension member comprises a support line. The support line has a first end and a second end. The second end of the support line is attached to the first end of an anchoring member. Fig. 3 shows an example of a support line 21 attached at its second end 23 to the first end 25 of an anchoring member 24. The anchoring member has a ladder-shaped structure 24. Fig. 4 is an example of a support line 31 attached at its second end to the first end of an anchoring member 32, wherein the anchoring member has a ladder-shaped structure connected to three retainers 33. In embodiments, the suspension member is implanted in the breast of the patient with the support line 31 positioned superior to the anchoring member. During implantation, a force is applied in an inferior to superior direction to the support line such that the retainers located on the anchoring member engage nearby breast tissue, and lift the breast tissue in an inferior to superior direction.

In embodiments, the suspension member's support line may have a length of 10 to 40 cm, and more preferably 15 to 25 cm.

In embodiments, the support line comprises a monofilament fiber, and more preferably a monofilament fiber with an average diameter of 0.1-0.4 mm, and more preferably 0.2-0.25 mm. In embodiments, the support line comprises a multifilament fiber. In embodiments, the support line comprises a braid.

In embodiments, the support line comprises fixation elements. The fixation elements are designed to engage breast tissue, and to help secure the support line in breast tissue after the suspension member has been used to lift the breast.

In embodiments, the fixation elements are connected to or incorporated into the support lines, and extend outwards from the support line.

In embodiments, the fixation elements are small filaments that extend from the support lines. The small filaments may be monofilaments or multifilaments.

In embodiments, the support lines with fixation elements are unitary structures. An example of a support line with fixation elements with a two-loop pillar stitch structure is shown in Detail A of Fig. 4 which is an expansion of area A of the support line 31. In other embodiments, the support line with fixation elements has a single loop pillar stitch structure.

In embodiments, the support lines with fixation elements are formed by textile processing. In other embodiments, the fixation elements are tied, fused or welded to the support lines. In embodiments, the support lines with fixation elements are molded.

In embodiments, the fixation elements comprise absorbable polymers. In embodiments, the fixation elements comprise one or more of the polymers listed in Section A.

In embodiments, the support line comprises an absorbable polymer. In embodiments, the support line comprises one or more polymers listed in Section A. Preferably, the support line comprises one or more of the following: poly-4-hydroxybutyrate or copolymer thereof, poly(butylene succinate) or copolymer thereof, or polydioxanone.

In embodiments, the suspension member comprises an anchoring member with a textile structure and retainers, and a support line connected at its second end to the first end of the anchoring member, and all components comprise the same polymer, preferably poly-4-hydroxybutyrate or copolymer thereof, poly(butylene succinate) or copolymer thereof, or polydioxanone.

### Anchoring Members

In embodiments, the suspension members 20 comprise anchoring members 24 (see Fig. 3). The anchoring members have a first end 25 and a second end 26. The anchoring members 24 are attached to a support line 21 at their first end 25. The anchoring member 24 is not connected to a support line at its second end 26. The second end is a free end. The anchoring member is not a sling or hammock. The anchoring member 24 does not have an enlarged 2D or 3D shape which is capable of spanning the lower pole of the breast. In contrast, an exemplary shape of the anchoring member is a band, ribbon or strip. In embodiments, the anchoring member has an appearance of an unrolled knit or string bracelet. Its length is much larger than its width.

The anchoring members are designed to engage breast tissue. After engagement of breast tissue, the suspension members can be used to lift the breast of a patient. Unlike sutures, the anchoring members contact the breast tissue over an extended area, which allows the anchoring members to spread the load applied during lifting of the breast over a large area of breast tissue. Spreading the load over an extended area helps to prevent the anchoring member from tearing the breast tissue, or applying too much tension to a localized region of the breast. The latter can result in an undesirable appearance of the breast. The anchoring members are particularly useful in lifting fatty breast tissue, unlike sutures which may cut through fatty breast tissue or not be retained by fatty tissue. In addition to providing an improved means for lifting breast tissue, the anchoring members provide a means to provide long term support of the lifted breast by creating an extended new tissue plane in the breast. The new tissue plane results from tissue in-growth into the anchoring member. The new tissue plane provides internal support for the lifted breast.

The anchoring members are preferably sized for delivery through a stab wound using an introducer tool. The anchoring members may be rolled or folded to allow delivery to the implantation site. The anchoring members may have shape memory allowing the anchoring members to be delivered to the implantation site with an introducer tool, and then resume their shape.

The anchoring members are designed to be inserted on either the lateral or medial side of the breast to lift breast tissue. Preferably, at least one anchoring member is inserted on the lateral side of the breast, and at least one anchoring member is inserted on the medial side of the breast. The entire breast may be lifted using an anchoring member on each side of the breast.

In embodiments, the suspension member's anchoring member may have a length of 8 to 20 cm, and more preferably 12 to 16 cm, and a width of 4 to 20 mm, and more preferably 6 to 12 mm. In a preferred embodiment, the anchoring member has a length to width ratio between 5 to 50, and more preferably between 10 to 25. Stated another way, the length is preferably 10 to 25 times greater than the width of the anchoring member.

The anchoring members preferably retain at least 15% of their initial strength, more preferably at least 30% of their initial strength, and even more preferably at least 50% of their initial strength for 12 weeks after implantation. The anchoring members preferably can withstand a burst force of at least 1 N, more preferably at least 10 N, but less than 1,000 N.

The anchoring members preferably comprise absorbable polymers, more preferably absorbable thermoplastic polymers, and even more preferably synthetic or biosynthetic absorbable thermoplastic polymers. In embodiments, the anchoring members comprise one or more of the following: poly-4-hydroxybutyrate or copolymer thereof, poly(butylene succinate) or copolymer thereof, or polydioxanone. In a preferred embodiment, the anchoring members are completely absorbable. In a particularly preferred embodiment, the anchoring members are transitory.

In embodiments, the anchoring members are porous.

The anchoring members preferably comprise textiles. Textiles include mesh, braids, woven mesh, and knitted mesh. These textiles preferably comprise monofilament fibers, and more preferably absorbable monofilament fibers. In a particularly preferred embodiment, the absorbable monofilament fibers are polymeric and oriented. Oriented in this embodiment means that the fibers have been stretched to increase the molecular alignment of polymer chains in the fibers. In embodiments, the monofilament fibers have average diameters of 0.02 to 0.7 mm, more preferably 0.05 to 0.25 mm, and even more preferably 0.07 to 0.175 mm. In embodiments, the anchoring member comprises a textile with one or more of the following properties: (i) a suture pullout strength of at least 1 Kgf, (ii) a burst strength of 0.1 to 100 Kg, (iii) a thickness of 0.05-5 mm, (iv) an areal density of 5 to 800 g/m², and (v) a pore diameter of 5 µm to 10 mm. In a preferred embodiment, the anchoring member of the implant comprises a textile, and the textile has one or more of the following properties: (i) a suture pullout strength of 1 Kgf to 20 Kgf, (ii) a burst strength of 1 to 50 Kg or 10 to 50 Kg, (iii) a thickness of 0.1 to 1 mm, (iv) an areal density of 100 to 300 g/m², and (v) a pore diameter of 100 µm to 1 mm.

In embodiments, the anchoring members comprise one or more of the polymers listed in Section A. These polymers may be used to prepare a textile structure of an anchoring member. Preferably, the anchoring members comprise one or more of the following: poly-4-hydroxybutyrate or copolymer thereof, or poly(butylene succinate) or copolymer thereof. In embodiments, the anchoring members comprise meshes prepared from one or more of the following: fibers comprising poly-4-hydroxybutyrate or copolymer thereof, poly(butylene succinate) or copolymer thereof, or polydioxanone.

In embodiments, the anchoring member comprises one or more bioactive agents. Bioactive agents may be coated on the anchoring members, or incorporated into the anchoring members. Particularly preferred bioactive agents include anti-microbials, antibiotics, proteins, and agents to promote tissue in-growth. In embodiments, the anchoring member comprises one or more of the following: cells, stem cells, gels, hydrogels, fatty tissue, autologous fat, fat lipoaspirate, adipose cells, and fibroblast cells. These cells and materials may be coated onto the anchoring members or incorporated into the anchoring members.

In embodiments, the anchoring members have a ladder-shape. In an embodiment, the suspension member comprises a ladder-shaped anchoring member. Fig. 3 is an example of a suspension member 20 comprising a ladder-shaped anchoring member 24 connected to the second end 23 of a support line 21. Preferably, the ladder-shaped anchoring member is knitted with fiber, and more preferably knitted with a monofilament fiber. In embodiments, the ladder-shaped anchoring member is knitted using a combination of interlocked pillar stitch to create the vertical members (i.e. the sides of the ladder), and a weft-inserted inlay to create the horizontal members (i.e. the steps) of the ladder. The pillar stitch and inlay may be formed with fiber of the same average diameter, or with fibers of different average diameters. In an embodiment, the anchoring member comprises a ladder-shaped knitted structure wherein the vertical members of the ladder are formed from a monofilament fiber with a smaller diameter than the monofilament fiber used to form the horizontal members of the ladder. In an embodiment, the vertical members of the ladder are formed with a monofilament fiber having an average diameter of 0.02-0.3 mm, and more preferably 0.07-0.1 mm, and the horizontal members of the ladder are formed with a monofilament fiber having an average diameter of 0.05 to 0.35 mm, and more preferably 0.1 to 0.15 mm. In an embodiment, the ladder-shaped knitted structure has a length of 10-20 cm, and has a width of 5-12 mm. Preferably, the suspension member comprising the ladder-shaped anchoring member is completely absorbable, and even more preferably the ladder-shaped anchoring member is produced from poly-4-hydroxybutyrate or copolymer thereof, poly(butylene succinate) or copolymer thereof, or polydioxanone.

In embodiments, the anchoring members comprise retainers that may engage breast tissue. In embodiments, the retainers are designed to be inserted into a ladder-shaped textile to form the anchoring member. The ladder-shaped textile may be formed as described herein. Fig. 4 is an example of a suspension member 30 comprising a ladder-shaped textile with three retainers 33 inserted into the ladder structure in order to form the anchoring member 32. The anchoring member is connected to the second end of a support line 31. As shown in Fig. 4, the tips of the retainers are angled away from the plane of the anchoring member (i.e. away from the plane of the ladder structure), and all point in the same direction, namely in the direction of the support line 31. The retainers are angled to engage tissue when a lifting force is applied to the suspension member in an inferior to superior direction.

In an embodiment, the retainers comprise a base section and a tip. Detail A of Fig. 5 is an expansion of area A of suspension member 40 with an anchoring member 44 comprising three retainers 47, and shows a retainer 50, the ladder-shaped structure of the anchoring member 51, the retainer's base section 52, the retainer's tip 53, and the retainer secured 54 in the crisscrossed ladder-shaped structure 51. In embodiments, the base section 52 of the retainer 50 is connected to the plane of the anchoring member, and the tip section of the retainer 53 extends away from the plane of the anchoring member. In embodiments, the retainers are connected to the plane of the anchoring member at an angle in order to position the tip of the retainers to engage and lift breast tissue. In embodiments, the retainers are positioned at an angle to the plane of the anchoring members, wherein the angle between: (i) the first end of the anchoring member and the vertex of the angle, and (ii) the tip of the retainer and the vertex of the angle, is less than 90 degrees, but greater than 0 degrees. In embodiments, the tips of the retainers are positioned pointing in a superior direction after implantation of the suspension member. In embodiments, the base section of the retainer is positioned inferior to the tip of the retainer after implantation.

In embodiments, the retainer comprises grooves that are used to secure the retainer to the ladder-shaped structure of an anchoring member. Fig. 6 is an example of a retainer 80 showing the retainer's base section 81, tip 82, and grooves 83 for securing the retainer in the ladder-shaped structure of an anchoring member. Detail A of Fig. 5 shows a retainer with grooves secured in a ladder-shaped structure of an anchoring member.

In embodiments, the retainers have planar structures, such retainer 80 shown in Fig. 6. In other embodiments, the retainers have non-planar structures. An example of a retainer with a non-planar structure is shown in Fig. 7. In this example, retainer 90 has an angled tine 93 emanating from a planar base section 91. The base section contains grooves 94 for securing the base section 91 in a ladder-shaped structure (as shown in Detail A of Fig. 5). The retainers 90 are secured in the ladder-shaped structure so that the tips 92 of the retainers are angled to point in the direction of the support line, and can engage breast tissue when the suspension member is lifted in an inferior to superior direction.

In embodiments, the anchoring members comprise retainers wherein the retainers extend 0.1 to 25 mm, more preferably 1 to 15 mm, and even more preferably 3 to 10 mm from the plane of the anchoring member to the tips of the retainers. The retainers are designed to engage tissue, and be able to lift tissue when a force is applied to the anchoring member in an inferior to superior direction. Examples of suitable retainers that can be incorporated into the anchoring members include: anchors, swivel anchors, hooks, darts, barbs, clasps, projections, extensions, bulges, protuberances, spurs, bumps, points, cogs, surface roughness, surface irregularities, and arrows. Preferably, the retainers have sharp tips that can engage breast tissue.

In embodiments, the anchoring members comprise multiple retainers to engage breast tissue. In embodiments, the anchoring members comprise 1, 2, 3, 4, 5, 6, 7, 8, 9, 10, 11, 12, 13, 14, 15, 16, 17, 18, 19, 20, or more retainers. Multiple retainers allow the anchoring members to spread the load of the lifted breast over a large area. Spreading the load of the lifted breast with the retainers over a larger area of the breast tissue provides a more secure method of lifting breast tissue, prevents high tension being placed on discrete areas of breast tissue, and helps to maintain the lift and prevent subsequent ptosis. The use of multiple retainers is particularly desirable for lifting fatty breast tissue or less dense breast tissue. These tissues are typically difficult to lift, for example, using suture or other devices that anchor at a single point in the breast, because application of tension at localized points can result in tearing of the tissue and pull through of the device.

In embodiments, the anchoring members comprise textiles, and the base sections of the retainers are connected to the textiles. In embodiments, the base sections of the retainers of the anchoring members are secured in absorbable monofilament textiles, and more preferably braided, knitted, or woven textiles of absorbable monofilament. In a particularly preferred embodiment, the plane of the anchoring members comprise textiles with one or more of the following properties: (i) a suture pullout strength of at least 1 Kgf, (ii) a burst strength of 0.1 to 100 Kg, (iii) a thickness of 0.05-5 mm, (iv) an areal density of 5 to 800 g/m², and (v) a pore diameter of 5 µm to 10 mm, and one, two, three or more retainers selected from the group comprising: anchors, swivel anchors, hooks, darts, barbs, clasps, projections, extensions, bulges, protuberances, spurs, bumps, points, cogs, surface roughness, surface irregularities, and arrows, inserted in the textiles.

In embodiments, the retainers comprise absorbable polymers. In embodiments, the retainers comprise one or more of the polymers listed in Section A. Preferably, the retainers comprise one or more of the following: poly-4-hydroxybutyrate or copolymer thereof, poly(butylene succinate) or copolymer thereof, or polydioxanone. In embodiments, the plane of the anchoring member comprises a textile structure, the anchoring member comprises retainers, and both the textile structure and the retainers comprise the same polymer, preferably poly-4-hydroxybutyrate or copolymer thereof, poly(butylene succinate) or copolymer thereof., or polydioxanone.

### Introducer Housing Tip

In embodiments, the suspension members further comprise an introducer housing tip. The introducer housing tip is preferably connected to the second end of the anchoring member. The introducer housing tip is designed for use with an introducer tool, and is designed to receive the distal end of an introducer tool. Insertion of the distal end of an introducer tool into the introducer housing tip provides a means for the surgeon to deliver the suspension member into the breast. After delivery of the suspension member to the implant site, the tool is disconnected from the introducer housing tip and removed from the breast leaving the suspension member implanted in the desired location. In embodiments, the introducer housing tip has a blunt driving tip, and is designed for blunt dissection of breast tissue. Insertion of the distal end of an introducer tool into the introducer housing tip in this embodiment allows the surgeon to make a defined channel in the breast for insertion of the suspension member by pushing the introducer housing tip with the introducer tool through breast tissue with a penetration force sufficient to penetrate breast tissue. After the defined channel in the breast is formed, the introducer tool may be withdrawn from the breast leaving the suspension member with its attached introducer housing tip implanted in the defined channel.

The introducer housing tip is designed to allow insertion of the distal tip of the introducer tool into the introducer housing tip, and subsequent removal of the distal tip from the introducer housing tip once the suspension member has been delivered to the desired location in the breast.

In embodiments, the introducer housing tip is sized to allow passage of the tip through a stab incision. In embodiments, the introducer housing tip is sized to allow passage through a channel created in the breast by blunt dissection for insertion of a suspension member.

In a preferred embodiment, the introducer housing tip is designed to allow blunt dissection of breast tissue to create a defined channel in the breast for the suspension member. Preferably, the introducer housing tip has a conical shape 48 as shown in Fig. 5. Preferably, the introducer housing tip has a blunt driving tip 62 for blunt dissection of breast tissue.

The introducer housing tip is sized to accommodate the distal tip of an introducer tool for delivery of the suspension member to the implant site.

In a preferred embodiment, the suspension member has a ladder-shaped anchoring member with a first end and a second end, and is connected at its first end to the second end of a support line, and is connected at its second end to an introducer housing tip.

An example of an introducer housing tip connected to the second end of an anchoring member is shown in Fig. 5. In this example, the introducer housing tip 48 is connected to an anchoring member 44 at its second end 46. The anchoring member comprises a ladder-shaped structure and three retainers 47, and the anchoring member is connected at its first end 45 to the second end 43 of a support line 41. Detail B of Fig. 5 is an expansion of area B of the suspension member 40, and shows the location 60 for inserting the distal end of the introducer tool in the introducer housing tip 61, and the conical-shaped tip 62 used to channel the suspension member into the patient's breast. The introducer housing tip 61 preferably has a tapered shape as shown in Detail B of Fig. 5 with a blunt tip 62 for blunt dissection of breast tissue.

In embodiments, the introducer housing tip 61 has an internal bore at location 60 for inserting the introducer tool. The internal bore is sized to accommodate the distal tip of the introducer tool. Preferably, the diameter of the internal bore is 1-4 mm, and more preferably 2 mm, and the length of the internal bore is preferably 0.5-2 cm, and more preferably 1 cm. In embodiments, the introducing housing tip has a maximum outside diameter of 2-10 mm, more preferably 3-8 mm, and even more preferably 4-6 mm. In embodiments, the length of the introducer housing tip is 10-30 mm, and more preferably 15 mm. In embodiments, the introducer housing tip has a conical shape 61, with a tapered shape ending in a blunt round tip 62.

Fig. 8 is a diagram showing how an introducer tool 100 with a straight needle 101 is inserted into the introducer housing tip 102. The introducer housing tip 102 is connected to anchoring member 103 of suspension member 104. Detail D in Fig. 8 is an expansion of area D, and shows the distal end of the introducer tool tip (105) inserted in the introducer housing tip 102, and the connection 106 of the introducer housing tip 102 to the ladder-shaped structure 107 of the anchoring member 103.

In embodiments, the introducer housing tip is 3D printed, or molded. Preferably, the introducer housing tip is injection molded.

In embodiments, the introducer housing tip is connected to the second end of the anchoring member by fusing the tip and anchoring member together. In embodiments, the introducer housing tip is connected to the second end of the anchoring member by ultrasonic welding. Fig. 5 shows the introducer housing tip ultrasonically welded to the ladder-shaped anchoring member.

In embodiments, the introducer housing tip is absorbable. Preferably, the introducer housing tip is resorbed after implantation. In embodiments, the introducer housing tip is formed from an absorbable polymer. In embodiments, the introducer housing tip comprises polymers listed in Section A. Preferably, the introducer housing tip comprises one or more of the following: poly-4-hydroxybutyrate or copolymer thereof, poly(butylene succinate) or copolymer thereof., or polydioxanone. In embodiments, the support line, anchoring member, retainers and introducer housing tip comprise the same absorbable polymer, and more preferably comprise poly-4-hydroxybutyrate or copolymer thereof, poly(butylene succinate) or copolymer thereof, or polydioxanone.

### Strut

In embodiments, a strut is used in conjunction with suspension members to lift the breast. Fig. 1 shows the implanted positions of two suspension members 3 and 4 in the breast of a patient, and their points of connection to an implanted strut 5 at connection points 6. The strut 5 is implanted superior to the patient's NAC. After tensioning of the suspension members 3 and 4 to lift the breast, the support lines of the suspension members are connected to strut 5 in order to maintain tension and the lifted position of the breast.

In embodiments, the strut has two arms with a central element. Figure 9 is a diagram of a suitable strut 110. The strut 110 has a first arm 111 having a first end 112 and a second end 113, a second arm 114 having a first end 115 and a second end 116, and an element 117 with a first end 118 and a second end 119, wherein the second end of the first arm is connected to the first end of the element, and the second end of the second arm is connected to the second end of the element. Preferably, the element 117 is a plate or textile. In embodiments, the plate may be a film. Detail F shown in Fig. 9 is an expansion of area F of element 117 showing perforations 120 in the element to encourage tissue ingrowth into the element 117. For example, the perforations may be made in a film to form element 117. Detail F in Fig. 9 also shows tines 121 that can be incorporated into element 117. For example, the tines may be formed on the surface of a film or injection molded plate to form element 117. The tines help to anchor the strut in the patient's tissue, and prevent movement of the strut after its implantation. In embodiments, the element 117 comprises perforations and tines. In embodiments, the strut 110 further comprises one or more needles. In embodiments, the strut 110 comprises two needles with a needle attached to each of the arms 111 and 114. Figure 9 shows a first needle 122 attached to the first end 112 of the first arm 111, and a second needle 123 attached to the first end 115 of the second arm 114. The first and second needles 122 and 123 preferably have blunt tips.

In embodiments, the arms 111 and 114 of the strut comprise fibers, either monofilament or multifilament fibers, but more preferably monofilament fibers. Even more preferably the arms comprise absorbable monofilament fibers or oriented absorbable monofilament fibers. In embodiments, the average diameters of the fibers are from 0.02 mm to 0.5 mm, more preferably 0.06 mm to 0.3 mm, and even more preferably from 0.1 mm to 0.2 mm. In embodiments, the arms comprise polymers listed in Section A. Preferably, the arms comprise one or more of the following: poly-4-hydroxybutyrate or copolymer thereof, poly(butylene succinate) or copolymer thereof, or polydioxanone.

In embodiments, the element 117 is a plate, injection molded plate, or film, and more preferably a perforated plate or film. In embodiments, the pores of the element have a diameter of at least 50 µm, and more preferably at least 75 µm. In embodiments, the pores of the element have average diameters between 75 µm and 3 mm, and more preferably 250 µm and 1 mm. In embodiments, the element has tines protruding from its surface. In embodiments, the tines protrude 0.1 to 3 mm from the surface of the element, and more preferably 0.5 to 2 mm. In embodiments, the element can be rolled up into a small tube for delivery to the implant site, and unrolled once delivered to the implant site.

In embodiments, the element 117 is a textile, preferably a knitted, braided or woven textile. In embodiments, the textile is porous, and has average pore sizes of at least 50 µm, more preferably at least 75 µm, and even more preferably between 75 µm and 3 mm, or 250 µm and 1 mm. In embodiments, the textile is designed so that it can be rolled up into a small tube for delivery to the implant site, and unrolled once delivered to the implant site.

In embodiments, the element 117 comprises polymers listed in Section A. Preferably, element 117 comprises one or more of the following: poly-4-hydroxybutyrate or copolymer thereof, poly(butylene succinate) or copolymer thereof, or polydioxanone. In embodiments, the support line, anchoring member, retainers, introducer housing tip, and strut comprise the same absorbable polymer, and more preferably comprise poly-4-hydroxybutyrate or copolymer thereof, poly(butylene succinate) or copolymer thereof, or polydioxanone.

In embodiments, the arms 111 and 114 are connected to element 117 by fusion, welding or tying. In embodiments, the arms 111 and 114 and element 117 are knit, woven, braided, molded or 3D printed as a unitary structure.

In embodiments, a strut is formed by injection molding an element 117 in the form of a plate and connecting the plate at its first and second ends to arms 111 and 114 by tying the arms to the plate. In embodiments, the arms are manually tied to the element 117 by knotting. In embodiments, the arms are 10-25 cm long, and more preferably 20 cm long. In embodiments, the arms have an average minimum diameter of 0.1 mm and an average maximum diameter of 0.3 mm. In embodiments, the arms 111 and 114 are formed from monofilament fiber. In embodiments, the monofilament fiber has an average minimum diameter of 0.1 mm and an average maximum diameter of 0.3 mm, and a length of 10-25 cm. In embodiments, the element 117, for example, an injection molded plate, is formed with dimensions of 2 cm x 5 cm x 0.5 mm (width x length x thickness). In embodiments, the injection molded plate further contains (a) conical shaped tines 121 (see Fig. 9, Detail F) with a height extending 0.5 mm from the surface of the plate, and a base diameter of 0.4 mm, and (b) pores 120 (see Fig. 9, Detail F) with a diameter of 0.8 mm. In embodiments, the density of the tines is 5 tines per cm², and the density of the pores is 16 pores per cm². In embodiments, the strut is formed with a needle attached to arm 111 or 114, or more preferably with a needle attached to each of the arms 111 and 114. Preferably, the needles have blunt ends. In embodiments, one arm is swaged to a needle, or both arms are swaged to needles. Figure 9 shows the first ends 112 and 115 of each arm 111 and 114 swaged to needles 122 and 123, respectively. In embodiments, the needles are straight. In embodiments, the needle size is 10-gauge. In embodiments, the injection molded element 117 and arms 111 and 114 made of monofilament fiber comprise the polymers listed in Section A. Preferably, the injection molded element 117 and the monofilament fiber comprise one or more of the following: poly-4-hydroxybutyrate or copolymer thereof, poly(butylene succinate) or copolymer thereof, or polydioxanone.

In embodiments, the strut is formed by knitting or weaving. In embodiments, the strut comprises an element 117 in the form of a textile that grips tissue. In an embodiment, the textile is a warp knitted mesh, and preferably is knit from monofilament fiber. The monofilament fiber preferably has an average minimum diameter of 0.05 mm to 0.2 mm, and more preferably an average minimum diameter of 0.07 mm to 0.15 mm. In embodiments, the element 117 is a self-gripping mesh. The self-gripping mesh may be formed, for example, by knitting a spacer mesh on a 20-guage double needle bed warp knitting machine using monofilament fiber, and using a hot knife to cut the monofilaments connecting the front and back meshes of the spacer mesh. Cutting the monofilaments produces mesh with exposed monofilament ends. These constructs can be further cut to the desired dimensions (width and length) of element 117, and have a density of monofilament ends exposed on one side of the mesh (i.e. monofilament tines protruding from the mesh on one side). The density of monofilament ends is preferably 10-50 per square cm. Preferably, the monofilament ends protrude away from the body of the mesh by 0.5-5 mm, and more preferably 1-3 mm. In an embodiment, an element 117 formed with a self-gripping mesh may be connected at its first and second ends to monofilament fiber by tying the fiber to the self-gripping mesh, to form the arms 111 and 114. In embodiments, arms 111 and 114 formed from monofilament fiber has an average minimum diameter of 0.2 mm and an average maximum diameter of 0.25 mm. In embodiments, the arms 111 and 114, and the element 117 may be knit as a unitary structure. In embodiments, the textile element 117 and monofilament fiber arms 111 and 114 comprise one or more of the polymers listed in Section A. Preferably, the textile element 117 and the monofilament fiber arms comprise one or more of the following: poly-4-hydroxybutyrate or copolymer thereof, poly(butylene succinate) or copolymer thereof, or polydioxanone.

### Tensioner

In embodiments, the suspension members further comprise a tensioner. Examples of tensioners include a pulley system, or a spool tensioner. In embodiments, the tensioner may be adjusted during implantation, or the tensioner may be adjusted after implantation, for example, to apply more tension to a support line or an anchoring member to lift the breast. In embodiments, the tensioner may be adjusted using a small tool that is inserted through a small incision.

In embodiments, the tensioner is preferably incorporated in the support line of the suspension member, but more preferably incorporated at the first or second ends of the support line.

In embodiments, the tensioner comprises an absorbable polymer. In embodiments, the tensioner comprises one or more polymers listed in Section A. Preferably, the tensioner comprises one or more of the following: poly-4-hydroxybutyrate or copolymer thereof, poly(butylene succinate) or copolymer thereof, or polydioxanone.

### Sheaths

In embodiments, the suspension member is partially or totally inserted inside a removable sheath for delivery of the suspension member to the implant site. In embodiments, the anchoring member of the suspension member is inserted inside a removable sheath. All or part of the suspension member may be inserted inside a sheath in order to facilitate delivery of the suspension member to the implant site. In particular, the removable sheath may be used to prevent the anchoring member or fixation elements from engaging breast tissue prematurely during implantation, and particularly before the suspension member is located in the desired position in the breast. In embodiments, a suspension member is partly or fully inserted inside a removable sheath, the sheath comprising the suspension member is implanted in the breast, and the removable sheath is removed to deploy the suspension member in the breast.

In embodiments, the removable sheath comprises a polymer. In embodiments, the sheath is formed from nylon, high density polyethylene, polytetrafluoroethylene and low density polyethylene.

### Introducer Tool

In embodiments, the suspension member comprises an introducer housing tip, and is designed to be implanted using an introducer tool that can be inserted in the introducing housing tip. For example, in location 60 of the introducer housing tip 61 (see Fig. 5, Detail B). In embodiments, the introducer tool 100 comprises a handle, a needle section 101 emanating from the handle, and a distal tip 105 (located at the end of the needle on the opposite end to the handle) that is designed to be inserted in the introducer housing tip at location 60. In embodiments, the handle is made from medical grade polypropylene. In embodiments, the handle is 10-15 cm, more preferably 12 cm, in length, and has a diameter of 2-3 cm, more preferably 2.5 cm. In embodiments, the introducer tool has a universal handle design to allow for both left- and right-handed use. In embodiments, the needle of the introducer tool is made from 316L stainless steel. In embodiments, the needle has a length of 20-30 cm, more preferably 25 cm. In embodiments, the needle has a diameter of 3 mm next to the handle that reduces to a diameter of 2 mm at the distal end of the introducer tool (at the end opposite to the handle, e.g. location 105), and more preferably reduces to a diameter of 2 mm at a distance of 1 cm from the distal end of the tool. The reduced diameter at the end of the needle is designed to allow insertion of the needle tip section in the introducer housing tip at location 60, and delivery of the suspension member to the implant site.

In embodiments, the introducer tool comprises a straight needle. In embodiments, the introducer tool comprises a curved needle.

### Mastopexy Systems

In embodiments, systems for mastopexy are provided. The systems may comprise implants and one or more tools for implantation of the implants. In a preferred embodiment, the systems comprise two suspension members, a strut, an introducer tool. The suspension members, strut and introducer tool may be prepared as described herein. The systems may further comprise one or more of the following: a stab incision tool and a blunt dissection tool. In embodiments, the systems may comprise two suspension members, a strut, and two introducer tools as shown, for example, in Fig. 2.

In embodiments, a mastopexy system for securing a patient's breast in a target position is provided, wherein the mastopexy system comprises: a first suspension member comprising a first support line with a first end and a second end, and a first anchoring member with a first end and a second end, the first support line connected at its second end to a first anchoring member at its first end; a second suspension member comprising a second support line with a first end and a second end, and a second anchoring member with a first end and a second end, the second support line connected at its second end to a second anchoring member at its first end, wherein one suspension member when implanted in the breast is located on the lateral side of the breast, and the second suspension member when implant in the breast is located on the medial side of the breast, such that the support lines of the suspension members are located superior to the anchoring members, and the first ends of the support lines are located above the nipple areolar complex (NAC). The mastopexy system may further comprise one or more of the following: a stab incision tool, a blunt dissection tool, and an introducer tool. The mastopexy system preferably further comprises introducer housing tips, designed to connect to an introducer tool, located on the second end of each of the suspension member's anchoring member. Introducer tools can be inserted into these introducer housing tips, and the assembly of the introducer tool and introducer housing tip used to create defined channels through the breast tissue for the suspension members by blunt dissection. After forming a defined channel in the breast, the introducer tool is removed from the breast leaving the suspension member, still connected to the introducer housing tip, implanted in the breast. Alternatively, the introducer tools can be inserted into the introducer housing tips, and used to insert the suspension members into channels already created in the breast by a blunt dissection tool. In order to facilitate delivery of the suspension members to the implant sites in the breast, the mastopexy system may further comprise suspension members partly or fully covered by removable sheaths. The sheaths are removed after the suspension members have been implanted in their desired locations in the breast. The sheaths prevent the anchoring members from engaging tissue prematurely. The mastopexy system may further comprise a strut that can be implanted in a position superior to the NAC of the breast. The strut preferably comprises a first arm with a first end and a second end, a second arm with a first end and a second end, and an element with a first end and a second end, wherein the second end of the first arm is connected to the first end of the element, and wherein the second end of the second arm is connected to the second end of the element. The arms of the strut are preferably formed from monofilament fiber, multifilament fiber or braided fiber. The element is preferably a plate, such as a film, or textile. The element is preferably porous, and preferably has a self-gripping feature on one side. The pores and self-gripping feature are designed to allow tissue in-growth into the element, and to secure the strut in place. The self-gripping feature may, for example, be tines, or short monofilament fibers. Preferably, the strut so configured may be implanted in the breast in a position superior to the NAC, and the breast lifted by connecting the first arm of the strut to a first end of a support line of a first suspension member, and connecting the second arm of the strut to a second end of a support line of a second suspension member. In embodiments, the strut of the mastopexy system is preferably inserted in a channel, superior to the NAC, that is formed by blunt dissection. Preferably, the channel and position of the implanted strut is along a medial-lateral plane superior to the NAC of the breast. If desired, the strut may be enclosed in a removable sheath to facilitate implantation, and the sheath removed once the strut is located in the breast in the desired position. After implantation of the suspension members and strut in the breast, and removal of any sheaths, tension may be applied to the support lines of the suspension members to lift the breast, and the support lines connected to the arms of the strut to maintain the breast in the lifted position.

An exemplary system for mastopexy consisting of a ladder-shaped knitted anchoring member with mounted retainers, connected at its first end to a support line made of a monofilament fiber with an average minimum diameter of 0.2 mm and an average maximum diameter of 0.25 mm, and connected by fusion at its second end to an introducer housing tip may be prepared as follows. The ladder-shaped structure of the anchoring member is prepared with a width of 8 mm, and a length of 14 cm using monofilament fibers. The ladder-shaped anchoring member is warp knitted, using a combination of an interlocked pillar stitch and monofilament fiber with a minimum average diameter of 0.07 mm and an average maximum diameter of 0.1 mm, to create the vertical rails of the ladder, and a weft-inserted inlay stitch made of monofilament fiber with a minimum average diameter of 0.1 mm and an average maximum diameter of 0.15 mm to create the horizontal members (i.e. the steps of the ladder). Three retainers, prepared by injection molding, are manually inserted using a crisscross pattern (see Fig. 5 Detail A) into the horizontal members of the knitted ladder-shaped anchoring member, and spaced 3 cm apart from each other on the ladder-shaped structure of the anchoring member. The retainers are molded with dimensions of 4 mm x 25 mm x 2 mm (width x length x thickness). The retainers are shaped with a base section (see 81 in Fig. 6) and five circular grooves of 1 mm diameter (see 83 in Fig. 6), set 4 mm apart on each side of the retainer, to secure the retainers in the ladder-shaped anchoring member (as shown in Detail A of Fig. 5). Each retainer is formed with a 30-degree angled tip (see feature 82 in Fig. 6). The support line, made from monofilament fiber is formed with a length of 20 cm, and is connected by tying to the first end of the ladder-shaped anchoring member. The second end of the ladder-shaped anchoring member is ultrasonically welded to an injection molded introducer housing tip. The introducer housing tip (see 61 in Fig. 5 Detail B) is formed with a conical shape with a maximum outside diameter at its base of 5 mm, a length of 15 mm, an internal bore at location 60 with a diameter of 2.1 mm and a length of 12.5 mm, and a tapered section with a 20 degree angle terminating in a blunt round tip 62. The exemplary mastopexy system further comprises a strut. The strut is formed by injection molding a plate with conical shaped tines and perforations (as shown in Detail F of Fig. 9) and tying monofilament fibers to either ends of the plate 117 to form arms 111 and 114, or by knitting element 117 of the strut from monofilament fiber and attaching monofilament fiber arms to either end of the element 117. The anchoring member with mounted retainers, monofilament support line, introducer housing tip, strut element and arms can be formed from one or more of the polymers listed in Section A. More preferably, these components are formed from poly-4-hydroxybutyrate or copolymer thereof, poly(butylene succinate) or copolymer thereof, or polydioxanone.

In embodiments, the systems for mastopexy are designed for minimally invasive delivery. The implants have a design and properties that allow them to be delivered through a small incision. In embodiments, the implants are designed so that they can be rolled or folded to allow delivery through a small incision. This minimally invasive approach can reduce patient morbidity, scarring and the chance of infection. In embodiments, the implants have shape memory properties that allow them to assume their original shape unaided after they has been delivered to the implant site. For example, the implant may be temporarily deformed by rolling it up into a small diameter cylindrical shape for delivery to the implant site, and then allowed to resume its original shape unaided *in vivo.*

### METHODS FOR IMPLANTING THE MASTOPEXY IMPLANTS

The implants and systems described herein are most suited for use in breast surgery, and more particularly for mastopexy procedures.

Figs. 10-13 show a method for performing a breast lift with a mastopexy implant. Fig. 10 shows an outline of a patient's breast 130, the breast's NAC 131, and an introducer tool 132 loaded with a first suspension member 133 (shown in more detail as implant 44 in Fig. 5) inserted on a first side of the patient's breast. The method preferably involves making a stab incision at location 134 shown in Fig. 10. The distal end of the introducer tool 132 is inserted in the introducer housing tip of the first suspension member (shown in more detail in Fig. 8). The introducer housing tip has a blunt driving tip designed for blunt dissection of breast tissue. The introducer housing tip is inserted in the stab incision, and advanced using the introducer tool to form a defined channel by blunt dissection in the side of the breast for the first suspension member by applying a force to the introducer tool in the direction of the arrow shown in Fig. 10. In embodiments, the first channel has a relatively small diameter, and is straight or linear shaped. The direction of the channel is from posterior to inferior, or vice versa, and laterally spaced from the NAC 151. In a sense, it is a one dimensional channel compared to a large 2-D planar type dissection required for a sling-type implant which spans the entire lower pole of the breast.

Once the first suspension member has been advanced to the desired location, the introducer tool 132 is removed from the breast leaving the implanted first suspension member in the breast. Fig. 11 shows the implanted first suspension member 142, and the implantation of a second suspension member 144 on the opposite side of the breast through stab incision location 146 using the same procedure that was used to insert the first suspension member. A second linear channel is created for the second suspension member. The second channel is shown spaced apart from the nipple. The first and second channels do not connect or overlap, and in embodiments, are substantially parallel to one another. Each channel is shown as a discrete linear narrow channel, and spaced laterally or medially from the NAC 151.

Once the second suspension member 144 has been advanced to the desired location using introducer tool 143, the introducer tool 143 is removed from the breast leaving the implanted first and second suspension members in the breast. The method shown in Figs. 10 and 11 may further comprise inserting the suspension members, partially or fully enclosed in sheaths, and removing the sheaths after implantation of the suspension members.

In contrast to some of the prior art sling-type devices described above, which span the lower pole from one side of the breast to the other side, the second ends of each of the suspension members are deployed in the tissue to the side of the NAC 151, and are free or unconnected to additional members once deployed.

In an alternative method to that shown in Figs. 10 and 11, the introducer tools 132 and 143 may have curved needles instead of straight needles, and be used to create curved channels in the breast spaced laterally or medially from the NAC 131 or 141. In this alternative method, the suspension members are implanted in the curved channels.

Fig. 12 is a diagram showing a method for insertion of a strut 155 (shown in more detail as 110 in Fig. 9) in the patient's breast. The strut 155 may be inserted through one of the stab incisions (e.g. 157) in the breast. In an embodiment, the strut is inserted into a defined channel already created in the breast between stab incision locations 156 and 157 by blunt dissection. Alternatively, the defined channel for the strut may be formed by blunt dissection at the same time the strut is implanted. In embodiments, a needle with a blunt tip attached to an arm of a strut, as shown in Fig. 9, may be used to insert the strut by blunt dissection. In embodiments, an introducer tool may be used to create a channel for the strut by driving a needle attached to an arm of the strut through breast tissue above the NAC. Fig. 13 is a diagram showing an implanted strut 165 located superior to the NAC 161 of the breast, and the introducer tool 164 used to implant the strut. The strut is preferably inserted in a medial to lateral plane as shown in Fig. 13. After implantation of the strut 165, the introducer 164 is withdrawn leaving the suspension members and strut implanted in the breast as shown in Fig. 1. The breast is lifted by applying tension in an inferior to superior direction to the support lines, and connecting the support lines to the arms of the strut 165. (Fig. 1 shows in more detail the connection of the support lines to the arms of the strut at connection points 6.) Any suitable method may be used to connect the support lines to the strut arms. In embodiments, the support lines are tied, stapled, or fused to the strut arms. In embodiments, excess support line and the arms of the strut may be trimmed after the support lines have been connected to the arms of the strut. Any needles attached to the arms of the strut are removed subsequent to implantation of the strut. In embodiments, tension is applied to the support lines, and the support lines are connected to the strut arms while the patient is in an upright position. While the method shown in Figs. 10-13 shows the implantation of one suspension member on each side of the breast, additional suspension members may be inserted in the breast, if desired, to provide the desired lift.

In an embodiment, a method of lifting a breast of a patient, comprises the steps of: (i) introducing a first suspension member comprising a first support line with a first end and a second end, and a first anchoring member with a first end and a second end, the first support line connected at its second end to a first anchoring member at its first end, on the lateral side of the breast, (ii) introducing a second suspension member comprising a second support line with a first end and a second end, and a second anchoring member with a first end and a second end, the second support line connected at its second end to a second anchoring member at its first end, on the medial side of the breast, and (iii) connecting the first end of the first support line to the first end of the second support line in a position superior to the NAC of the breast, fixating the first end of the first support line and the first end of the second support line to posture tissue in one or more positions superior to the NAC of the breast, or introducing a strut superior to the NAC of the breast, and connecting the first ends of the first and second support lines to the strut. In embodiments, the anchoring members are configured with retainers to engage and lift tissue. In embodiments, the support lines are configured with fixation elements to engage tissue. In embodiments, the posture tissue is selected from one or more of the following: muscle, pectoral muscle, intercostal tissue, fascia, bone, rib, collar bone, ligament, tendon and skin. In embodiments, the method involves inserting the suspension members in the breast in a superior to inferior direction. In embodiments, the method involves inserting the suspension members in the breast in an inferior to superior direction. In embodiments, the anchoring members further comprise introducer housing tips at the second ends of the anchoring members. In embodiments, the method comprises inserting an introducer tool in the introducer housing tips connected to the anchoring members and using the introducer tool to implant the suspension members in the breast. In embodiments, the introducer housing tips have blunt driving tips, and are used to form defined channels in the breast for implantation of the suspension members. In embodiments, defined channels are formed in the breast by using an introducer tool to push the introducer housing tip with a blunt driving tip through breast tissue with a penetration force sufficient to penetrate breast tissue. After each channel is formed, the introducer tool is removed from the breast leaving the suspension member connected to the introducer housing tip implanted in the breast. In embodiments, the introducer housing tips have conical shapes with blunt driving tips to facilitate the formation of the defined channels in the breast. In embodiments, the strut comprises a first arm with a first end and a second end, a second arm with a first end and a second end, and a textile or plate with a first end and a second end, wherein the second end of the first arm is connected to the first end of the textile or plate, wherein the second end of the second arm is connected to the second end of the textile or plate, and wherein the method of lifting the breast comprises connecting the first end of the first support line to the first arm of the strut and connecting the first end of the second support line to the second arm of the strut after implantation of the strut and suspension members in the breast. In embodiments, the method further comprises one or more of the following steps: (a) making one or more stab incisions in the breast, (b) inserting an introducer housing tip connected to a suspension member, or a blunt dissection tool, in the stab incision, (c) creating straight or curved channels on the medial and lateral sides of the breast by blunt dissection for insertion of suspension members, (d) inserting a suspension member with an introducer tool, and implanting the suspension member in the breast, (e) inserting a suspension member in the breast wherein the suspension member is partially or completely covered by a sheath, and removing the sheath from the breast after insertion of the suspension member in the breast, (f) applying tension to one or more of the suspension members in an inferior to superior direction to lift the breast, optionally after sitting the patient in an upright position, (g) trimming the support lines after connecting the support lines together, (h) creating a straight or curved channel superior to the NAC of the breast by blunt dissection for insertion of the strut.

In an embodiment, a method of lifting a breast comprises: providing a stab incision tool, an introducer tool, a suspension member comprising a support line with a first end and a second end, and an anchoring member with a first end and a second end, the support line connected at its second end to the anchoring member at its first end, wherein an introducer housing tip with a blunt dissection tip is connected to the second end of the anchoring member, making a stab incision in the breast, connecting the introducer tool to the introducer housing tip, and inserting the introducer housing tip in the stab incision, making a defined channel in the breast, on the lateral or medial side of the breast, by using the introducer tool to push the introducer housing tip through breast tissue with a penetration force sufficient to penetrate breast tissue and to deliver the suspension member connected to the introducer housing tip into the channel, with the support line of the suspension member in a superior position to the anchoring member, applying a force to the support line to lift the breast, and securing the support line in a position superior to the NAC of the breast. In an embodiment, the method further comprises using a blunt dissection tool to form a channel in a medial to lateral direction above the NAC of the breast, inserting a strut in the channel, and connecting a support line to the strut to secure the lifted breast in an elevated position.

Modifications and variations of the methods and compositions will be apparent from the foregoing detailed description, the scope of the present invention being defined by the wording of the appended claims.

## Claims

1. A mastopexy system, comprising:
a suspension member (3; 4; 10; 11; 20; 30; 40; 104; 133; 142; 144; 152; 153; 162; 163) comprising a support line (21; 31; 41; 70) with a first end (22; 42) and a second end (23; 43), and an anchoring member (24; 44; 46; 51; 103) with a first end (25) and a second end (26), the support line connected at its second end to the anchoring member at its first end, and wherein the anchoring member is only connected to a support line at one end; and
a strut (5; 12; 110; 155; 165) configured to attach to the support line,
**characterized in that** the anchoring member comprises a ladder-shaped textile.

2. The mastopexy system of claim 1, further comprising an introducer housing tip (48; 61; 102) connected to the second end (26) of the anchoring member (24; 44; 46; 51; 103), the introducer housing tip having a conical shape with a blunt end at the apex of the conical shape, and a bore for inserting a distal tip of an introducer tool (13; 100; 105; 132; 143; 154; 164; 167).

3. The mastopexy system of claim 1, further comprising at least one retainer (33; 47; 50; 80; 90), and wherein the anchoring member (24; 44; 46; 51; 103) is configured with the retainers to engage and lift tissue.

4. The mastopexy system of claim 3, wherein the retainers (33; 47; 50; 80; 90) are selected from one or more of the following: anchors, swivel anchors, hooks, darts, barbs, clasps, projections, extensions, bulges, protuberances, spurs, bumps, points, cogs, surface roughness, surface irregularities, and arrows.

5. The mastopexy system of any one of claims 3-4, wherein the retainers (33; 47; 50; 80; 90) are positioned at an angle on the anchoring members (24; 44; 46; 51; 103), and the retainers are angled at an angle of less than 90 degrees measured between (i) the first end (25) of the anchoring member and the vertex of the angle and (ii) the tip of the retainer and the vertex of the angle.

6. The mastopexy system of claim 1, wherein the support line (21; 31; 41; 70) is configured with fixation elements (34) to fixate the support line in soft tissue.

7. The mastopexy system of claim 6, wherein the fixation elements (34) are selected from one or more of the following: braids, warp knits, and warp knits comprising pillar stitches.

8. The mastopexy system of claim 1, wherein the suspension member (3; 4; 10; 11; 20; 30; 40; 104; 133; 142; 144; 152; 153; 162; 163) can withstand a load of at least 5 N, or wherein the anchoring member (24; 44; 46; 51; 103) can withstand a burst force of at least 1 N.

9. The mastopexy system of claim 1, wherein the suspension member (3; 4; 10; 11; 20; 30; 40; 104; 133; 142; 144; 152; 153; 162; 163) is partially or totally inserted in a removable sheath.

10. The mastopexy system of any one of claims 1 to 9, wherein the strut (5; 12; 110; 155; 165) comprises a first arm (111), a second arm (114) and a textile or plate connected to the first arm and the second arm, wherein the first arm is configured to attach to the support line (21; 31; 41; 70).

11. The mastopexy system of any one of claims 1 to 10, wherein the suspension member (3; 4; 10; 11; 20; 30; 40; 104; 133; 142; 144; 152; 153; 162; 163) is
a first suspension member, wherein the support line (21; 31; 41; 70) is a first support line, wherein the anchoring member (24; 44; 46; 51; 103) is a first anchoring member, the system further comprising:
a second suspension member comprising a second support line with a first end (22; 42) and a second end (23; 43), and a second anchoring member with a first end (25) and a second end, the second support line connected at its second end to a second anchoring member at its first end; and
wherein the strut (5; 12; 110; 155; 165) is configured to connect to the first suspension member and the second suspension member;
wherein one suspension member when implanted in the breast is located on the lateral side of the breast, and the second suspension member when implanted in the breast is located on the medial side of the breast, such that the support lines of the suspension members are located superior to the anchoring members, and the first ends of the support lines are located above the NAC.

12. The mastopexy system of claim 11, wherein the strut (5; 12; 110; 155; 165) provides a means for attachment of the strut to the support lines (21; 31; 41; 70).

13. The mastopexy system of claim 12, wherein the strut (5; 12; 110; 155; 165) when implanted in the breast is located superior to the NAC of the breast.

14. The mastopexy systems of any one of claims 12 or 13, wherein the strut (5; 12; 110; 155; 165) comprises a first arm (111) with a first end (112) and a second end (113), a second arm (114) with a first (116) end and a second end (116), and a textile or plate with a first end and a second end, wherein the second end of the first arm is connected to the first end of the textile or plate, and wherein the second end of the second arm is connected to the second end of the textile or plate, and optionally wherein a needle is connected to the first end of one of the first and second arms arm or wherein needles are connected to the first ends of each of the first and second arms.

15. A method of making the mastopexy system of any one of claims 1 or 11, comprising connecting the first end of the ladder-shaped textile to the second end of the support line (21; 31; 41; 70), the support line preferably either being formed with fixation elements (34) that can engage tissue, or by knitting a textile with a single or double loop pillar stitch.

16. The method of claim 15, wherein
the ladder-shaped textile is formed by knitting a monofilament fiber,
the support line (21; 31; 41; 70) preferably being a monofilament fiber which is optionally connected to the anchoring member (24; 44; 46; 51; 103) by tying, stapling, or fusion.

## Patentansprüche

1. Mastopexiesystem, umfassend:
ein Aufhängungselement (3; 4; 10; 11; 20; 30; 40; 104; 133; 142; 144; 152; 153; 162; 163), umfassend ein Tragseil (21; 31; 41; 70) mit einem ersten Ende (22; 42) und einem zweiten Ende (23; 43) und ein Verankerungselement (24; 44; 46; 51; 103) mit einem ersten Ende (25) und einem zweiten Ende (26), wobei das Tragseil an seinem zweiten Ende mit dem Verankerungselement an seinem ersten Ende verbunden ist, und wobei das Verankerungselement nur an einem Ende mit einem Tragseil verbunden ist; und
eine Strebe (5; 12; 110; 155; 165), die zur Befestigung an dem Tragseil konfiguriert ist,
**dadurch gekennzeichnet, dass** das Verankerungselement ein leiterförmiges Textil umfasst.

2. Mastopexiesystem nach Anspruch 1, weiter umfassend eine Einführungsgehäusespitze (48; 61; 102), die mit dem zweiten Ende (26) des Verankerungselements (24; 44; 46; 51; 103) verbunden ist, wobei die Einführungsgehäusespitze eine konische Form mit einem stumpfen Ende an der Spitze der konischen Form und eine Bohrung zum Einführen einer distalen Spitze eines Einführungswerkzeugs (13; 100; 105; 132; 143; 154; 164; 167) aufweist.

3. Mastopexiesystem nach Anspruch 1, weiter umfassend mindestens einen Halter (33; 47; 50; 80; 90), und wobei das Verankerungselement (24; 44; 46; 51; 103) mit den Haltern so konfiguriert ist, dass es Gewebe erfasst und anhebt.

4. Mastopexiesystem nach Anspruch 3, wobei die Halter (33; 47; 50; 80; 90) aus einem oder mehreren der folgenden ausgewählt sind: Anker, Wirbelanker, Haken, Pfeile, Widerhaken, Klammern, Vorsprünge, Verlängerungen, Ausbuchtungen, Vorsprünge, Sporen, Beulen, Spitzen, Zähne, Oberflächenrauheit, Oberflächenunregelmäßigkeiten und Pfeile.

5. Mastopexiesystem nach einem der Ansprüche 3-4, wobei die Halter (33; 47; 50; 80; 90) in einem Winkel an den Verankerungselementen (24; 44; 46; 51; 103) positioniert sind, und die Halter in einem Winkel von weniger als 90 Grad abgewinkelt sind, gemessen zwischen (i) dem ersten Ende (25) des Verankerungselements und dem Scheitelpunkt des Winkels, und (ii) der Spitze des Halters und dem Scheitelpunkt des Winkels.

6. Mastopexiesystem nach Anspruch 1, wobei das Tragseil (21; 31; 41; 70) mit Fixierungselementen (34) konfiguriert ist, um das Tragseil in Weichgewebe zu fixieren.

7. Mastopexiesystem nach Anspruch 6, wobei die Fixierungselemente (34) aus einem oder mehreren der folgenden ausgewählt sind: Geflechte, Kettengewirke und Kettengewirke, umfassend Fransenmaschen.

8. Mastopexiesystem nach Anspruch 1, wobei das Aufhängungselement (3; 4; 10; 11; 20; 30; 40; 104; 133; 142; 144; 152; 153; 162; 163) einer Belastung von mindestens 5 N standhalten kann, oder wobei das Verankerungselement (24; 44; 46; 51; 103) einer Berstkraft von mindestens 1 N standhalten kann.

9. Mastopexiesystem nach Anspruch 1, wobei das Aufhängungselement (3; 4; 10; 11; 20; 30; 40; 104; 133; 142; 144; 152; 153; 162; 163) teilweise oder vollständig in eine abnehmbare Hülle eingesetzt ist.

10. Mastopexiesystem nach einem der Ansprüche 1 bis 9, wobei die Strebe (5; 12; 110; 155; 165) einen ersten Arm (111), einen zweiten Arm (114) und ein Textil oder eine Platte umfasst, verbunden mit dem ersten Arm und dem zweiten Arm, wobei der erste Arm zum Befestigen an dem Tragseil (21; 31; 41; 70) konfiguriert ist.

11. Mastopexiesystem nach einem der Ansprüche 1 bis 10, wobei das Aufhängungselement (3; 4; 10; 11; 20; 30; 40; 104; 133; 142; 144; 152; 153; 162; 163)
ein erstes Aufhängungselement ist, wobei das Tragseil (21; 31; 41; 70) ein erstes Tragseil ist, wobei das Verankerungselement (24; 44; 46; 51; 103) ein erstes Verankerungselement ist, wobei das System weiter Folgendes umfasst:
ein zweites Aufhängungselement, umfassend ein zweites Tragseil mit einem ersten Ende (22; 42) und einem zweiten Ende (23; 43), sowie ein zweites Verankerungselement mit einem ersten Ende (25) und einem zweiten Ende, wobei das zweite Tragseil an seinem zweiten Ende mit einem zweiten Verankerungselement an seinem ersten Ende verbunden ist; und
wobei die Strebe (5; 12; 110; 155; 165) so konfiguriert ist, dass sie mit dem ersten Aufhängungselement und dem zweiten Aufhängungselement verbunden wird;
wobei sich ein Aufhängungselement bei Implantation in die Brust an der lateralen Seite der Brust befindet, und sich das zweite Aufhängungselement bei Implantation in die Brust an der medialen Seite der Brust befindet, so dass sich die Tragseile der Aufhängungselemente über den Verankerungselementen befinden, und die ersten Enden der Tragseile oberhalb der NAC liegen.

12. Mastopexiesystem nach Anspruch 11, wobei die Strebe (5; 12; 110; 155; 165) ein Mittel zur Befestigung der Strebe an den Tragseilen (21; 31; 41; 70) bereitstellt.

13. Mastopexiesystem nach Anspruch 12, wobei sich die Strebe (5; 12; 110; 155; 165) bei Implantation in die Brust oberhalb der NAC der Brust befindet.

14. Mastopexiesysteme nach einem der Ansprüche 12 oder 13, wobei die Strebe (5; 12; 110; 155; 165) einen ersten Arm (111) mit einem ersten Ende (112) und einem zweiten Ende (113), einen zweiten Arm (114) mit einem ersten (116) Ende und einem zweiten Ende (116), und ein Textil oder eine Platte mit einem ersten Ende und einem zweiten Ende umfasst, wobei das zweite Ende des ersten Arms mit dem ersten Ende des Textils oder der Platte verbunden ist, und wobei das zweite Ende des zweiten Arms mit dem zweiten Ende des Textils oder der Platte verbunden ist, und optional wobei eine Nadel mit dem ersten Ende eines von dem ersten und dem zweiten Arm verbunden ist, oder wobei Nadeln mit den ersten Enden jedes von dem ersten und dem zweiten Arm verbunden sind.

15. Verfahren zum Herstellen des Mastopexiesystems nach einem der Ansprüche 1 oder 11, umfassend das Verbinden des ersten Endes des leiterförmigen Textils mit dem zweiten Ende des Tragseils (21; 31; 41; 70), wobei das Tragseil bevorzugt entweder mit Fixierungselementen (34) ausgebildet ist, die in Gewebe eingreifen können, oder durch Stricken eines Textils mit einer einfachen oder doppelten Maschenreihe hergestellt ist.

16. Verfahren nach Anspruch 15, wobei
das leiterförmige Textil durch Stricken einer Monofilamentfaser gebildet wird,
wobei das Tragseil (21; 31; 41; 70) bevorzugt eine Monofilamentfaser ist, die wahlweise durch Verknoten, Klammern, oder Verschmelzen mit dem Verankerungselement (24; 44; 46; 51; 103) verbunden ist.

## Revendications

1. Système de mastopexie, comprenant :
un élément de suspension (3 ; 4 ; 10 ; 11 ; 20 ; 30 ; 40 ; 104 ; 133 ; 142 ; 144 ; 152 ; 153 ; 162 ; 163) comprenant une ligne de support (21 ; 31 ; 41 ; 70) avec une première extrémité (22 ; 42) et une seconde extrémité (23 ; 43), et un élément d'ancrage (24 ; 44 ; 46 ; 51 ; 103) avec une première extrémité (25) et une seconde extrémité (26), la ligne de support étant connectée à sa seconde extrémité à l'élément d'ancrage à sa première extrémité, et dans lequel l'élément d'ancrage est uniquement connecté à une ligne de support à une extrémité ; et
une entretoise (5 ; 12 ; 110 ; 155 ; 165) configurée pour s'attacher à la ligne de support,
**caractérisé en ce que** l'élément d'ancrage comprend un textile en forme d'échelle.

2. Système de mastopexie selon la revendication 1, comprenant en outre un embout de boîtier d'introduction (48 ; 61 ; 102) connecté à la seconde extrémité (26) de l'élément d'ancrage (24 ; 44 ; 46 ; 51 ; 103), l'embout de boîtier d'introduction présentant une forme conique avec une extrémité arrondie au sommet de la forme conique, et un alésage pour insérer l'embout distal d'un outil d'introduction (13 ; 100 ; 105 ; 132 ; 143 ; 154 ; 164 ; 167).

3. Système de mastopexie selon la revendication 1, comprenant en outre au moins un dispositif de retenue (33 ; 47 ; 50 ; 80 ; 90), et dans lequel l'élément d'ancrage (24 ; 44 ; 46 ; 51 ; 103) est configuré avec les dispositifs de retenue pour venir en prise et soulever un tissu.

4. Système de mastopexie selon la revendication 3, dans lequel les dispositifs de retenue (33 ; 47 ; 50 ; 80 ; 90) sont sélectionnés parmi un ou plusieurs des éléments suivants : ancrages, ancrages pivotants, crochets, fléchettes, picots, agrafes, saillies, extensions, renflements, protubérances, éperons, butées, pointes, dents, rugosités de surface, irrégularités de surface et flèches.

5. Système de mastopexie selon l'une quelconque des revendications 3 à 4, dans lequel les dispositifs de retenue (33 ; 47 ; 50 ; 80 ; 90) sont positionnés selon un angle sur les éléments d'ancrage (24 ; 44 ; 46 ; 51 ; 103), et les dispositifs de retenue sont inclinés selon un angle inférieur à 90 degrés mesuré entre (i) la première extrémité (25) de l'élément d'ancrage et le sommet de l'angle et (ii) l'embout du dispositif de retenue et le sommet de l'angle.

6. Système de mastopexie selon la revendication 1, dans lequel la ligne de support (21 ; 31 ; 41 ; 70) est configurée avec des éléments de fixation (34) pour fixer la ligne de support dans des tissus mous.

7. Système de mastopexie selon la revendication 6, dans lequel les éléments de fixation (34) sont sélectionnés parmi un ou plusieurs des éléments suivants : tresses, tricots chaîne et tricots chaîne comprenant des chaînettes.

8. Système de mastopexie selon la revendication 1, dans lequel l'élément de suspension (3 ; 4 ; 10 ; 11 ; 20 ; 30 ; 40 ; 104 ; 133 ; 142 ; 144 ; 152 ; 153 ; 162 ; 163) peut résister à une charge d'au moins 5 N, ou dans lequel l'élément d'ancrage (24 ; 44 ; 46 ; 51 ; 103) peut résister à une force d'éclatement d'au moins 1 N.

9. Système de mastopexie selon la revendication 1, dans lequel l'élément de suspension (3 ; 4 ; 10 ; 11 ; 20 ; 30 ; 40 ; 104 ; 133 ; 142 ; 144 ; 152 ; 153 ; 162 ; 163) est partiellement ou totalement inséré dans une gaine amovible.

10. Système de mastopexie selon l'une quelconque des revendications 1 à 9, dans lequel l'entretoise (5 ; 12 ; 110 ; 155 ; 165) comprend un premier bras (111), un second bras (114) et un textile ou une plaque connecté(e) au premier bras et au second bras, dans lequel le premier bras est configuré pour s'attacher à la ligne de support (21 ; 31 ; 41 ; 70).

11. Système de mastopexie selon l'une quelconque des revendications 1 à 10, dans lequel l'élément de suspension (3 ; 4 ; 10 ; 11 ; 20 ; 30 ; 40 ; 104 ; 133 ; 142 ; 144 ; 152 ; 153 ; 162 ; 163) est
un premier élément de suspension, dans lequel la ligne de support (21 ; 31 ; 41 ; 70) est une première ligne de support, dans lequel l'élément d'ancrage (24 ; 44 ; 46 ; 51 ; 103) est un premier élément d'ancrage, le système comprenant en outre :
un second élément de suspension comprenant une seconde ligne de support avec une première extrémité (22 ; 42) et une seconde extrémité (23 ; 43), et un second élément d'ancrage avec une première extrémité (25) et une seconde extrémité, la seconde ligne de support étant connectée à sa seconde extrémité à un second élément d'ancrage à sa première extrémité ; et
dans lequel l'entretoise (5 ; 12 ; 110 ; 155 ; 165) est configurée pour se connecter au premier élément de suspension et au second élément de suspension ;
dans lequel un élément de suspension implanté dans le sein est situé sur le côté latéral du sein, et le second élément de suspension implanté dans le sein est situé sur le côté médial du sein, de sorte que les lignes de support des éléments de suspension soient situées au-dessus des éléments d'ancrage et que les premières extrémités des lignes de support soient situées au-dessus du NAC.

12. Système de mastopexie selon la revendication 11, dans lequel l'entretoise (5 ; 12 ; 110 ; 155 ; 165) fournit un moyen pour attacher l'entretoise aux lignes de support (21 ; 31 ; 41 ; 70).

13. Système de mastopexie selon la revendication 12, dans lequel l'entretoise (5 ; 12 ; 110 ; 155 ; 165), lorsqu'elle est implantée dans le sein, est située au-dessus du NAC du sein.

14. Systèmes de mastopexie selon l'une quelconque des revendications 12 ou 13, dans lesquels l'entretoise (5 ; 12 ; 110 ; 155 ; 165) comprend un premier bras (111) avec une première extrémité (112) et une seconde extrémité (113), un second bras (114) avec une première (116) extrémité et une seconde extrémité (116), et un textile ou une plaque avec une première extrémité et une seconde extrémité, dans lesquels la seconde extrémité du premier bras est connectée à la première extrémité du textile ou de la plaque, et dans lesquels la seconde extrémité du second bras est connectée à la seconde extrémité du textile ou de la plaque, et facultativement dans lesquels une aiguille est connectée à la première extrémité d'un des premier et second bras ou dans lesquels des aiguilles sont connectées aux premières extrémités de chacun des premier et second bras.

15. Procédé de fabrication du système de mastopexie selon l'une quelconque des revendications 1 ou 11, comprenant la connexion de la première extrémité du textile en forme d'échelle à la seconde extrémité de la ligne de support (21 ; 31 ; 41 ; 70), la ligne de support étant de préférence soit formée d'éléments de fixation (34) qui peuvent venir en prise avec du tissu, soit par tricotage d'un textile avec une chaînette à boucle simple ou double.

16. Procédé selon la revendication 15, dans lequel
le textile en forme d'échelle est formé par tricotage d'une fibre monofilament,
la ligne de support (21 ; 31 ; 41 ; 70) étant de préférence une fibre monofilament qui est facultativement connectée à l'élément d'ancrage (24 ; 44 ; 46 ; 51 ; 103) par ligature, agrafage ou fusion.
